# EUROPÄISCHE PATENTSCHRIFT

(11) **EP 4 218 859 B1**
(45) Veröffentlichungstag und Bekanntmachung des Hinweises auf die Patenterteilung: **17.06.2026**
(21) Anmeldenummer: 23155397.5
(22) Anmeldetag: 16.12.2020
(51) Int. Cl.: A61M 5/20, A61M 5/315, A61M 5/32, A61M 5/24

(54) **INJEKTIONSEINRICHTUNG ZUM INJIZIEREN EINER PHARMAZEUTISCHEN SUBSTANZ, UND INJEKTIONSLADETEIL FÜR EINE SOLCHE INJEKTIONSEINRICHTUNG**
INJECTION DEVICE FOR INJECTING A PHARMACEUTICAL SUBSTANCE, AND INJECTION LOADING PART FOR SUCH AN INJECTION DEVICE
DISPOSITIF D'INJECTION POUR INJECTER UNE SUBSTANCE PHARMACEUTIQUE ET PIÈCE DE CHARGEMENT À INJECTION POUR UN TEL DISPOSITIF D'INJECTION

(30) Priorität: 19.12.2019 DE 102019220296
(43) Veröffentlichungstag der Anmeldung: 02.08.2023
(62) Teilanmeldung aus: 20839258.9
(73) Patentinhaber: Vetter Pharma-Fertigung GmbH & Co. KG, 88212 Ravensburg (DE)
(72) Erfinder: NAUMBURG, Torsten, 88339 Bad Waldsee-Untermöllenbronn (DE); LIMBECK, Roland, 88400 Biberach (DE)
(74) Vertreter: Kordel, Mattias

(56) Entgegenhaltungen:
- EP-A1- 2 583 704
- EP-A2- 2 742 962
- DE-A1- 102006 041 809
- US-A1- 2015 045 729

## Beschreibung

Die Erfindung betrifft eine Injektionseinrichtung zum Injizieren einer pharmazeutischen Substanz sowie ein Injektionsladeteil zur Verwendung in einer solchen Injektionseinrichtung.

Injektionseinrichtungen der hier angesprochenen Art dienen zum insbesondere einmaligen oder wiederholten Injizieren einer pharmazeutischen Substanz. In besonders einfacher Ausgestaltung als herkömmlicher Pen können sie eine Antriebseinrichtung aufweisen, an der auswechselbar eine Karpule befestigt werden kann. Mit der Karpule kann wiederum eine Injektionsnadel verbunden werden. Durch die Antriebseinrichtung kann ein Kolben in der Karpule verlagert werden, um eine Injektion durchzuführen und die pharmazeutische Substanz über die Injektionsnadel auszutreiben. Dabei ist die Injektionsnadel bei der Verwendung des Pens für einen Anwender sichtbar. Dies kann zu psychischen und/oder physischen Abwehrreaktionen führen und letztlich die Compliance mit einem Behandlungsplan negativ beeinflussen. Ein solcher Pen kann wiederverwendet werden, indem eine entleerte Karpule von der Antriebseinrichtung getrennt und durch eine frische Karpule ersetzt wird. Es ist auch möglich, dass ein Pen zur einmaligen Injektion vorgesehen ist, wobei der gesamte Pen einschließlich der Antriebseinrichtung nach der Injektion entsorgt wird. Ein solcher Pen ist integral aufgebaut. Er ist daher nicht an verschiedene Primärverpackungen für pharmazeutische Substanzen adaptierbar beziehungsweise muss für jede Anwendung neu entwickelt werden. Darüber hinaus ist die Verwendung eines solchen Einmal-Pens teuer und kommt daher nur für hochpreisige pharmazeutische Substanzen überhaupt in Betracht. Mit dem Bayer BETACONNECT^{®} ist eine Injektionseinrichtung bekannt geworden, in die eine spezifisch ausgestaltete Spritze geladen werden kann. Die Injektionseinrichtung kann mehrfach verwendet werden, und sie weist einen Sichtschutz auf, sodass eine Injektionsnadel der in die Injektionseinrichtung geladenen Spritze für einen Anwender während der Anwendung verborgen bleibt. Die Injektionseinrichtung ist relativ kompliziert zu bedienen, wobei die Spritze insbesondere durch Aufklappen und seitliches Einlegen in das Gerät eingebracht werden muss; außerdem ist diese Injektionseinrichtung - wie ausgeführt - zur Verwendung mit einer bestimmten Art von Spritze eingerichtet und daher nicht mit anderen Primärpackmitteln verwendbar. US 2015/045729 A1 beschreibt eine Injektionseinrichtung gemäß dem Stand der Technik.

Der Erfindung liegt die Aufgabe zugrunde, eine Injektionseinrichtung sowie ein Injektionsladeteil für eine solche Injektionseinrichtung zu schaffen, wobei die genannten Nachteile nicht auftreten.

Die Aufgabe wird gelöst, indem eine Injektionseinrichtung gemäß Anspruch 1 geschaffen wird. Weitere Ausgestaltungen sind in den abhängigen Ansprüchen beschrieben.

Die Injektionseinrichtung ist eingerichtet zum Injizieren einer pharmazeutischen Substanz. Die Injektionseinrichtung weist ein Proximalteil auf, das einen Antrieb aufweist, der eingerichtet ist, um die Injektion zu bewirken. Die Injektionseinrichtung weist außerdem ein Distalteil auf, das eingerichtet ist zur Aufnahme eines Injektionsladeteils, das ein Packmittelaufnahmeteil und ein in dem Packmittelaufnahmeteil gehaltenes Primärpackmittel aufweist. In dem Primärpackmittel ist eine pharmazeutische Substanz aufgenommen, wobei die pharmazeutische Substanz aus dem Injektionsladeteil injizierbar ist, wenn das Injektionsladeteil in dem Distalteil angeordnet ist. Das Distalteil ist dabei so ausgebildet, dass das Injektionsladeteil axial in das Distalteil eingebracht werden kann. Die Injektionseinrichtung weist einen Sichtschutz auf, der so ausgebildet und angeordnet ist, dass eine Injektionsnadel des Injektionsladeteils während einer Anwendung der Injektionseinrichtung für einen Anwender verborgen ist. Die Injektionseinrichtung weist mit dem Proximalteil und dem Distalteil einen einfachen und insbesondere klar strukturierten Aufbau auf: Insbesondere ist eine Funktionstrennung zwischen dem Proximalteil und dem Distalteil vorgesehen, wobei der Antrieb in dem Proximalteil vorgesehen ist, und wobei das Distalteil der Aufnahme des Injektionsladeteils dient. Dadurch kann das Distalteil im Vergleich zu dem Proximalteil einfach und kostengünstig ausgestaltet sein, was letztlich auch den Gesamtaufwand und die Gesamtkosten bei der Herstellung der Injektionseinrichtung senkt. Je nach konkreter Ausgestaltung kann darüber hinaus auf ein für sich genommen bekanntes Proximalteil, das heißt eine Antriebseinrichtung, eines herkömmlichen Pens zurückgegriffen werden, der um das Distalteil ergänzt wird, sodass die Bereitstellung der Injektionseinrichtung besonders einfach und mit geringem Entwicklungs- und/oder Herstellungsaufwand verbunden ist. Darüber hinaus ist die Injektionseinrichtung einfach zu bedienen und auch für den Anwender in ihrer Funktion leicht zu verstehen.

Dass das Distalteil eingerichtet ist zur Aufnahme des Injektionsladeteils, das seinerseits wiederum das Primärpackmittel aufnimmt, bringt es vorteilhaft mit sich, dass die Injektionseinrichtung in einfacher Weise auf verschiedene Primärpackmittel adaptiert werden kann. Hierzu bedarf es lediglich einer Anpassung des Injektionsladeteils auf das entsprechende Primärpackmittel derart, dass einerseits das Primärpackmittel in dem Injektionsladeteil aufgenommen werden kann, wobei andererseits das Injektionsladeteil in dem Distalteil aufgenommen werden kann. Die Injektionseinrichtung ist also flexibel auf verschiedene Anwendungen anpassbar.

Die Injektionseinrichtung, insbesondere das Distalteil und das Proximalteil, ist in bevorzugter Ausgestaltung zum wiederholten Injizieren einer pharmazeutischen Substanz eingerichtet, wobei sie als Mehrweg-Injektionseinrichtung und insbesondere nachladbar ausgebildet sein kann. Insbesondere sind das Distalteil und das Proximalteil als mehrfach verwendbare Teile ausgebildet. Ein verbrauchtes Injektionsladeteil kann dann in einfacher Weise durch ein frisches Injektionsladeteil ersetzt werden. Das Injektionsladeteil ist bevorzugt als Einwegteil ausgestaltet.

Die Injektionseinrichtung kann aber auch zum einmaligen Injizieren einer pharmazeutischen Substanz verwendet werden. Sie kann dann insbesondere gemeinsam mit einem verbrauchten Injektionsladeteil entsorgt werden. Es verbleibt gleichwohl der Vorteil, dass aufgrund des mehrteiligen Aufbaus mit dem Proximalteil, dem Distalteil und dem Injektionsladeteil eine einfache Anpassung an verschiedene Primärpackmittel und somit verschiedene Einsatzoptionen möglich ist.

Der Sichtschutz ermöglicht es in vorteilhafter Weise, dass der Anwender der Injektionseinrichtung die Injektionsnadel nicht zu Gesicht bekommt, sodass damit verbundene physische und/oder psychische Abwehrreaktionen vermieden werden. Daher ist auch eine Compliance im Zusammenhang mit der Verwendung der Injektionseinrichtung hoch.

Der Sichtschutz ist zugleich ein Stichschutz, sodass die Injektionsnadel für den Anwender während der Anwendung der Injektionseinrichtung abseits der eigentlich vorzunehmenden Injektion nicht nur optisch sondern auch haptisch verborgen bleibt. Dies vermeidet ungewollte Stichverletzungen abseits der eigentlich vorzunehmenden Injektion bei der Anwendung der Injektionseinrichtung.

Unter einem Sichtschutz wird insbesondere eine Einrichtung oder ein Element verstanden, das/die eingerichtet und angeordnet ist, um die Injektionsnadel insbesondere vor dem Blick, vorzugsweise aber auch vor einer ungewollten Berührung durch den Anwender zu verbergen.

Dass die Injektionsnadel während der Anwendung der Injektionseinrichtung für den Anwender verborgen ist, bedeutet insbesondere, dass die Injektionsnadel während der Durchführung der Injektion, vorzugsweise während eines Ladens des Injektionsladeteils in das Distalteil und während der Injektion, besonders bevorzugt zusätzlich auch während eines Entladens des Injektionsladeteils, vor den Blicken sowie bevorzugt auch vor einer ungewollten Berührung des Anwenders verborgen ist.

Der Sichtschutz verhindert dabei in bevorzugter Ausgestaltung nicht, dass ein Injektionsfortschritt oder Füllstand des Primärpackmittels durch den Anwender beobachtet werden kann. Insbesondere ist bevorzugt in einer Umfangswandung des Distalteils ein Sichtfenster ausgebildet, durch welches der Anwender, insbesondere während der Durchführung einer Injektion, den Injektionsfortschritt und/oder den Füllstand des Primärpackmittels beobachten kann.

Unter einer pharmazeutischen Substanz ist insbesondere ein Stoff oder Stoffgemisch zu verstehen, welches eine pharmazeutische oder medizinische Wirkung zeitigt, und/oder welcher/welches zu einem pharmazeutischen und/oder medizinischen Zweck injiziert wird. Eine solche pharmazeutische Substanz kann insbesondere wenigstens einen Wirk- und/oder Hilfsstoff, insbesondere wenigstens ein Medikament aufweisen.

Mit "proximal" wird hier und im Folgenden insbesondere eine Richtung im Koordinatensystem der Injektionseinrichtung und/oder des Injektionsladeteils bezeichnet, welche bestimmungsgemäß einer Injektionsstelle, an der eine Injektion mit der Injektionseinrichtung durchgeführt werden soll, abgewandt ist. Mit "distal" wird entsprechend eine Richtung im Koordinatensystem der Injektionseinrichtung und/oder des Injektionsladeteils bezeichnet, die bestimmungsgemäß der Injektionsstelle zugewandt ist. Entsprechend ist das Proximalteil der Injektionseinrichtung bei bestimmungsgemäßer Anwendung derselben der Injektionsstelle abgewandt, wobei das Distalteil bei bestimmungsgemäßer Anwendung der Injektionsstelle zugewandt ist.

Das Proximalteil ist insbesondere ein Backend der Injektionseinrichtung. Insbesondere kann es sich dabei auch um ein konventionelles, für sich genommen bekanntes Backend eines Injektionspens handeln.

Das Distalteil ist insbesondere ein Frontend der Injektionseinrichtung.

Das Distalteil ist mit dem Proximalteil mechanisch verbindbar oder verbunden. Es ist in bevorzugter Ausgestaltung möglich, dass das Distalteil einteilig mit dem Proximalteil ausgebildet ist.

Der Antrieb ist als elektrischer Antrieb ausgebildet. Bevorzugt ist der elektrische Antrieb eingerichtet, um die Injektion automatisch zu bewirken. In bevorzugter Ausgestaltung weist das Proximalteil ein Steuergerät auf, das eingerichtet ist, um den Antrieb zum Bewirken der Injektion anzusteuern.

Dass der Antrieb eingerichtet ist, um die Injektion zu bewirken, bedeutet insbesondere, dass durch Aktivieren des Antriebs ein Austreiben der pharmazeutischen Substanz und damit eine Injektion kausal verursacht werden kann.

Das Injektionsladeteil ist insbesondere ein in das Distalteil einsetzbares und bevorzugt aus dem Distalteil entnehmbares Teil, welches das Primärpackmittel und damit die pharmazeutische Substanz trägt. Insbesondere ist es bevorzugt möglich, dass das Injektionsladeteil austauschbar in dem Distalteil aufgenommen werden kann.

Das Primärpackmittel ist insbesondere eingerichtet, um unmittelbar die pharmazeutische Substanz in seinem Inneren aufzunehmen. Insbesondere ist das Primärpackmittel bevorzugt als Spritze oder als Karpule ausgebildet. Vorzugsweise trägt das Primärpackmittel die Injektionsnadel, die insbesondere an einem distalen Ende des Primärpackmittels angeordnet, insbesondere lösbar oder unlösbar mit dem Primärpackmittel verbunden sein kann.

Dass Distalteil weist bevorzugt eine ausgezeichnete Erstreckungsrichtung auf, wobei seine Erstreckung in der ausgezeichneten Erstreckungsrichtung länger ist als die Erstreckung in den beiden senkrecht auf der ausgezeichneten Erstreckungsrichtung stehenden Richtungen. Diese ausgezeichnete Erstreckungsrichtung wird als Axialrichtung bezeichnet. Das Distalteil ist also insbesondere entlang der Axialrichtung langgestreckt ausgebildet. Der Begriff "axial" bezieht sich insbesondere auf diese Axialrichtung. Eine Umfangsrichtung umgreift die Axialrichtung konzentrisch. Eine radiale Richtung steht senkrecht auf der Axialrichtung.

Dass das Injektionsladeteil axial in das Distalteil eingebracht werden kann, bedeutet bevorzugt insbesondere, dass es in axialer Richtung, insbesondere stirnseitig, in das Distalteil eingebracht, und bevorzugt ebenso aus dem Distalteil entnommen werden kann.

Gemäß einer Weiterbildung der Erfindung ist vorgesehen, dass die Injektionseinrichtung den Antrieb des Proximalteils als einzigen Antrieb aufweist. Es ist insbesondere vorzugsweise kein weiterer Antrieb vorgesehen, unabhängig von einem möglichen Zweck dieses weiteren Antriebs. Der Antrieb des Proximalteils wirkt vorzugsweise auf eine Kolbenstange der Injektionseinrichtung, um die Kolbenstange in axialer Richtung zu verlagern, insbesondere um die pharmazeutische Substanz aus dem Injektionsladeteil auszutreiben, und um - in Gegenrichtung - die Kolbenstange nach der Durchführung der Injektion zurückzuverlagern. Die Injektionseinrichtung ist vorzugsweise so eingerichtet, dass wenigstens eine andere mechanische Funktion der Injektionseinrichtung durch die Verlagerung der Kolbenstange bewirkt wird. Der Antrieb dient somit neben dem eigentlichen Bewirken der Injektion mindestens einer weiteren Funktionalität. Dies stellt eine besonders wirtschaftliche und einfache Ausgestaltung der Injektionseinrichtung dar.

Die wenigstens eine weitere mechanische Funktion ist vorzugsweise ausgewählt aus einer Gruppe, bestehend aus: Einem Einstechen der Injektionsnadel in die Injektionsstelle unter dem Sichtschutz; eine Verriegelung des Injektionsladeteils und/oder des Sichtschutzes, insbesondere in einer Injektionsstellung zum Bewirken der Injektion oder in einer Ruhestellung, um eine ungewollte Injektion zu verhindern; und einer Entriegelung des Injektionsladeteils und/oder des Sichtschutzes, insbesondere um ein Einstechen der Injektionsnadel in die Injektionsstelle zu ermöglichen.

Gemäß einer Weiterbildung der Erfindung ist vorgesehen, dass die Injektionseinrichtung als Pen oder als Autoinjektor ausgebildet ist. Somit ist die Injektionseinrichtung auf eine Weise ausgebildet, die von anderen, herkömmlichen Injektionseinrichtungen bereits bekannt ist, wobei auch deren Bedienung grundsätzlich bekannt ist. Ein Anwender wird daher keine größeren Schwierigkeiten haben, die Injektionseinrichtung anzuwenden. Insbesondere ist die Injektionseinrichtung eingerichtet zur Selbstinjektion der pharmazeutischen Substanz durch einen Patienten, sodass es zur Verwendung der Injektionseinrichtung vorteilhaft keines medizinischen Fachpersonals bedarf.

Die nachfolgend offenbarten Weiterbildungen und bevorzugten Ausgestaltungen bilden einerseits die Erfindung unabhängig voneinander weiter; andererseits bilden sie aber bevorzugt in Kombination miteinander ein nicht zur Erfindung gehörendes Beispiel:
Gemäß einer Weiterbildung der Erfindung ist vorgesehen, dass das Distalteil lösbar mit dem Proximalteil verbunden ist. Vorteilhaft können auf diese Weise das Distalteil und das Proximalteil unabhängig voneinander gehandelt und gehandhabt werden. Insbesondere ist es auf diese Weise möglich, zur Bereitstellung der Injektionseinrichtung ein herkömmliches Backend für einen herkömmlichen Pen zu verwenden, und die Injektionseinrichtung dadurch bereitzustellen, dass das für sich genommen bekannte Backend mit dem erfindungsgemäß ausgestalteten Distalteil verbunden wird. Dabei kann das Distalteil insbesondere anstelle einer herkömmlicherweise mit dem Backend zu verbindenden Karpule mit diesem verbunden werden. Selbstverständlich ist es aber auch möglich, dass auch das Proximalteil eigens für die erfindungsgemäße Injektionseinrichtung ausgelegt und bereitgestellt sind.

Ist das Distalteil lösbar mit dem Proximalteil verbunden, kann es insbesondere von dem Distalteil getrennt werden. Eine solche Trennung des Distalteils von dem Proximalteil kann insbesondere vorgesehen sein zum Einbringen des Injektionsladeteils in das Distalteil, und/oder zu Wartungs- oder Reparaturzwecken.

Die Injektionseinrichtung weist bevorzugt einen Verbindungsmechanismus auf, der eingerichtet ist, um das Distalteil lösbar mit dem Proximalteil zu verbinden. Der Verbindungsmechanismus kann gemäß einer bevorzugten Ausgestaltung als Steck-/Dreh-Verbindung, insbesondere als Bajonettverschluss, ausgebildet sein.

Es ist alternativ aber auch möglich, dass das Distalteil unlösbar mit dem Proximalteil verbunden ist. Dabei bedeutet der Begriff "unlösbar" insbesondere, dass das Distalteil nicht zerstörungsfrei oder jedenfalls nicht ohne Gewalteinwirkung von dem Proximalteil getrennt werden kann.

Gemäß einem nicht erfindungsgemäßen Beispiel ist vorgesehen, dass der Sichtschutz starr an dem Distalteil vorgesehen oder angeordnet ist. Der Sichtschutz ist in diesem Fall relativ zu dem Distalteil nicht verlagerbar. Bevorzugt ist der Sichtschutz einstückig, vorzugsweise materialeinheitlich, mit dem Distalteil ausgebildet. In besonders bevorzugter Ausgestaltung ist der Sichtschutz durch das Distalteil gebildet. Dies bedeutet insbesondere, dass das Distalteil selbst zumindest bereichsweise den Sichtschutz darstellt. In besonders einfacher und funktionssicherer Weise bleibt dabei die Injektionsnadel durch das Distalteil dem Blick des Anwenders verborgen, wobei das Distalteil bevorzugt zugleich verhindert, dass der Anwender die Injektionsnadel in unbeabsichtigter Weise berührt. Das Distalteil dient also zugleich auch als Stichschutz.

Gemäß einem nicht erfindungsgemäßen Beispiel ist vorgesehen, dass eine proximale Stirnseite des Distalteils eine Ladeöffnung aufweist, durch die das Injektionsladeteil in das Distalteil eingebracht werden kann. Die Ladeöffnung ist also so angeordnet und eingerichtet, dass sie ein Einführen des Injektionsladeteils in das Distalteil erlaubt. Das Injektionsladeteil wird dabei quasi von einer der Injektionsstelle bestimmungsgemäß abgewandten Seite in das Distalteil eingebracht. In Analogie zu der begrifflichen Beschreibung von Schusswaffen ist somit die Injektionseinrichtung gemäß diesem Ausführungsbeispiel als Hinterlader ausgebildet.

Um das Injektionsladeteil in das Distalteil einbringen zu können, wird das Distalteil bevorzugt von dem Proximalteil gelöst, sodass die proximale Stirnseite des Distalteils zugänglich ist. Bei dieser Ausgestaltung ist demnach bevorzugt das Distalteil lösbar mit dem Proximalteil verbunden.

Gemäß einem nicht erfindungsgemäßen Beispiel ist vorgesehen, dass in dem Distalteil eine Federeinrichtung angeordnet ist, an der das Injektionsladeteil anliegt, und gegen dessen Federkraft das Injektionsladeteil in dem Distalteil in distaler Richtung zur Durchführung einer Injektion verlagert werden kann, wenn das Injektionsladeteil in dem Distalteil angeordnet ist. Zur Durchführung einer Injektion wird somit das Injektionsladeteil innerhalb des Distalteils und relativ zu dem Distalteil in Richtung der Injektionsstelle verlagert. In bevorzugter Ausgestaltung wird zum Durchführen der Injektion eine distale Stirnseite des Distalteils im Bereich der Injektionsstelle auf die Haut eines Patienten aufgesetzt, und das Injektionsladeteil wird innerhalb des Distalteils und relativ zu diesem in distaler Richtung verlagert, sodass die Injektionsnadel in die Haut des Patienten eindringt, und die Injektion durchgeführt werden kann. Anschließend wird das Injektionsladeteil durch die Federkraft der Federeinrichtung wieder zurückgedrängt, sodass auch die Injektionsnadel wieder vollständig innerhalb des Distalteils angeordnet ist. Das Distalteil dient dabei als Sichtschutz, vorzugsweise zugleich als Stichschutz.

Die Federeinrichtung stützt sich bevorzugt mit einem distalen Ende an einer Schulter des Distalteils ab, während an einem proximalen Ende der Federeinrichtung das Injektionsladeteil anliegt. Die Federeinrichtung ist in bevorzugter Ausgestaltung als Feder, insbesondere als Schraubenfeder, ausgebildet.

Die Verlagerung des Injektionsladeteils innerhalb des Distalteils wird bevorzugt durch die Kolbenstange des Proximalteils und damit zugleich durch dessen Antrieb bewirkt.

Insbesondere ist das Injektionsladeteil in dem Distalteil zwischen einer Schutzstellung, in der die Injektionsnadel vollständig innerhalb des Distalteils angeordnet ist, und einer Injektionsstellung, in welcher die Injektionsnadel über die distale Stirnseite des Distalteils vorsteht, und in der die Injektion durchgeführt wird, verlagerbar. Zur Durchführung der Injektion wird also das Injektionsladeteil aus der Schutzstellung heraus in axialer Richtung entgegen der Federkraft der Federeinrichtung in die Injektionsstellung verlagert. Nach dem Durchführen der Injektion, insbesondere beim Zurückziehen der Kolbenstange, drängt die Federeinrichtung das Injektionsladeteil wieder zurück aus der Injektionsstellung in die Schutzstellung.

Gemäß einem nicht erfindungsgemäßen Beispiel ist vorgesehen, dass die Injektionseinrichtung ein Verriegelungsteil aufweist, das durch die Ladeöffnung des Distalteils in das Distalteil einführbar und eingerichtet ist, um an einer proximalen Anlagefläche des Injektionsladeteils anzuliegen. Das Verriegelungsteil weist einen Grundkörper und wenigstens ein an dem Grundkörper schwenkbar angelenktes Riegelelement auf. Das Verriegelungsteil ist innerhalb des Distalteils insbesondere zwischen einer ersten Axialposition, auch als Entriegelungsstellung bezeichnet, und einer zweiten Axialposition, auch als Verriegelungsstellung bezeichnet, verlagerbar. In der ersten Axialposition ist das Verriegelungsteil entfernt von einer Riegelöffnung angeordnet, die in einer Umfangswandung des Distalteils ausgebildet ist. In der zweiten Axialposition ist das Verriegelungsteil auf Höhe der Riegelöffnung angeordnet. Ist das Verriegelungsteil in der ersten Axialposition angeordnet, ist das Riegelelement in den Grundkörper eingeschwenkt. Ist das Verriegelungsteil in der zweiten Axialposition angeordnet, kann das Riegelelement ausgeschwenkt werden und insbesondere die Riegelöffnung durchgreifen, um so eine axiale Verlagerung des Injektionsladeteils - relativ zu dem Distalteil - zu sperren. Dadurch wird vorteilhaft zum einen ein zu tiefes Eindringen der Injektionsnadel in den Körper des Patienten verhindert, sofern dies nicht bereits durch die Federeinrichtung, insbesondere ein Auf-Block-Fahren der Federeinrichtung, verhindert wird; zum anderen wird aber auch verhindert, dass das Injektionsladeteil durch die Kraft der Federeinrichtung während der Injektion zu weit in Richtung der Schutzstellung zurückverschoben wird, wodurch sonst gegebenenfalls die Injektionsnadel nicht tief genug in den Körper des Patienten eindringen würde, oder ganz aus der Injektionsstelle herausgleiten könnte. Vorteilhaft verhindert die Verriegelung durch das Riegelelement so bevorzugt eine axiale Verlagerung des Injektionsladeteils während der Durchführung der Injektion sowohl in distaler als auch in proximaler Richtung. Dabei ist es möglich, dass das Riegelelement und die Riegelöffnung so aufeinander abgestimmt sind, dass eine bestimmte Toleranz bezüglich einer axialen Verlagerung des Injektionsladeteils innerhalb des Distalteils gegeben ist, wobei die Riegelöffnung insbesondere axiale Anschläge - sowohl in proximaler als auch in distaler Richtung - für das Riegelelement bereitstellt. Eine mögliche axiale Verlagerung des Injektionsladeteils während der Injektion wird somit durch die Riegelöffnung begrenzt.

Vorzugsweise ist das wenigstens eine Riegelelement L-förmig ausgebildet. Insbesondere weist das L-förmige Riegelelement zwei miteinander verbundene, vorzugsweise einstückig und/oder materialeinheitlich miteinander ausgebildete Schenkel auf, wobei es bevorzugt in einem Verbindungsbereich der beiden Schenkel gelenkig an dem Grundkörper des Verriegelungsteils angelenkt ist.

Das Verriegelungsteil ist bevorzugt an dem Proximalteil befestigt. In vorteilhafter Weise kann es so nicht verlorengehen, wenn das Distalteil von dem Proximalteil getrennt wird, insbesondere um das Injektionsladeteil in das Distalteil einzubringen. Es ist aber auch möglich, dass das Verriegelungsteil als separates Element vorliegt. Um ein Injektionsladeteil in das Distalteil einzubringen, wird in diesem Fall das Verriegelungsteil bevorzugt manuell aus dem Distalteil entnommen; das Injektionsladeteil wird in das Distalteil eingebracht, und anschließend wird das Verriegelungsteil wieder - ebenfalls über die proximale Ladeöffnung - in das Distalteil eingeführt.

Vorzugsweise sind an dem Grundkörper des Verriegelungsteils eine Mehrzahl von Riegelelementen schwenkbar angelenkt, in besonders bevorzugter Ausgestaltung zwei Riegelelemente. Die Riegelelemente sind bevorzugt in Umfangsrichtung gesehen gleichmäßig, das heißt in gleichen Winkelabständen voneinander, an dem Grundkörper angelenkt. Sind zwei Riegelelemente vorgesehen, liegen sich diese also bevorzugt diametral gegenüber. In der Umfangswandung des Distalteils sind bevorzugt mindestens so viele Riegelöffnungen ausgebildet, wie das Verriegelungsteil Riegelelemente aufweist, bevorzugt genau so viele, wobei die Lage der Riegelöffnungen in Umfangsrichtung gesehen bevorzugt auf die Lage der Riegelelemente abgestimmt ist, sodass die Riegelelemente in der Verriegelungsstellung in die Riegelöffnungen eingreifen können. In besonders bevorzugter Ausgestaltung sind in der Umfangswandung des Distalteils zwei einander diametral gegenüberliegende Riegelöffnungen vorgesehen.

Gemäß einem nicht erfindungsgemäßen Beispiel weist das Verriegelungsteil eine axiale Durchgriffsöffnung für die Kolbenstange auf, wobei das Verriegelungsteil so ausgebildet ist, dass das wenigstens eine Riegelelement in der ersten Axialposition in die Durchgriffsöffnung eingreift, sodass die Durchgriffsöffnung für die Kolbenstange gesperrt und das Verriegelungsteil von der Kolbenstange verlagerbar ist - insbesondere gemeinsam mit dem Injektionsladeteil und damit zugleich dem Primärpackmittel -, wobei das Riegelelement in der zweiten Axialposition die Durchgriffsöffnung freigibt, sodass die Kolbenstange durch die Durchgriffsöffnung hindurchgreifen und relativ zu dem Verriegelungsteil verlagert werden kann, insbesondere um einen Kolben des Primärpackmittels innerhalb des Primärpackmittels und relativ zu der Injektionsnadel zu verlagern, sodass die pharmazeutische Substanz aus dem Primärpackmittel ausgetrieben wird. Das Riegelelement stellt also in der Entriegelungsstellung einen Anschlag für die Kolbenstange bereit, sodass die Kolbenstange das Verriegelungsteil und das Injektionsladeteil gemeinsam in distaler Richtung verlagern kann, bis das Verriegelungsteil in die zweite Axialposition und damit in die Verriegelungsstellung gelangt. Nun kann das Riegelelement ausschwenken und wird insbesondere durch die Kolbenstange so verschwenkt, dass es die Riegelöffnung durchgreift, wobei es zugleich die Durchgriffsöffnung freigibt, sodass die Kolbenstange an dem Riegelelement vorbei das Verriegelungsteil durchgreifen und auf den Kolben des Primärpackmittels wirken kann. Insbesondere greift das L-förmige Riegelelement mit einem Innenschenkel seiner beiden Schenkel in der Entriegelungsstellung in die Durchgriffsöffnung ein, sodass die Kolbenstange an dem Innenschenkel anschlägt. Gelangt das Verriegelungsteil in den Bereich der Riegelöffnung, kann ein Außenschenkel der beiden Schenkel die Riegelöffnung durchgreifen, wobei zugleich der Innenschenkel aus der Durchgriffsöffnung ausschwenkt und somit den Weg für die Kolbenstange durch die Durchgriffsöffnung hindurch freigibt. Wird die Kolbenstange nach vollendeter Injektion aus der Durchgriffsöffnung zurückgezogen, wird das Verriegelungsteil durch die Kraft der Federeinrichtung in proximale Richtung gedrängt, wobei das Riegelelement wieder in den Grundkörper einschwenken kann. Somit durchgreift es dann nicht mehr die Riegelöffnung, und das Verriegelungsteil kann gemeinsam mit dem Injektionsladeteil in proximaler Richtung verlagert werden.

Hier enden diejenigen Weiterbildungen und bevorzugten Ausgestaltungen, die bevorzugt in Kombination miteinander ein nicht zur Erfindung gehörendes Beispiel bilden.

Die nachfolgend offenbarten Weiterbildungen und bevorzugten Ausgestaltungen bilden einerseits die Erfindung unabhängig voneinander weiter; andererseits bilden sie aber bevorzugt in Kombination miteinander ein bevorzugtes Ausführungsbeispiel der Erfindung:
Gemäß einer Weiterbildung der Erfindung ist vorgesehen, dass der Sichtschutz verlagerbar an dem Distalteil gehalten ist. Insbesondere ist der Sichtschutz zwischen einer Ruhestellung und einer Expositionsstellung verlagerbar, wobei er in der Ruhestellung die Injektionsnadel verbirgt, solange keine Injektion durchgeführt wird, wobei er in der Expositionsstellung die Injektionsnadel für die Injektion freistellt. Der Sichtschutz ist in axialer Richtung verlagerbar. Vorzugsweise weist der Sichtschutz einen bevorzugt radialen Vorsprung an seiner äußeren Umfangsfläche auf, mit dem er in einer äußersten Expositionsstellung, das heißt bei weitest möglicher Verlagerung in proximaler Richtung, an einer distalen Stirnfläche des Distalteils oder an einem verstellbaren Anschlag anschlägt. Auf diese Weise wird vorteilhaft die Verlagerung des Sichtschutzes in proximaler Richtung begrenzt.

Bevorzugt ist der Sichtschutz in die Ruhestellung vorgespannt, wobei er gegen die Vorspannung aus der Ruhestellung in die Expositionsstellung verlagerbar ist. Hierfür weist das Distalteil bevorzugt eine Vorspanneinrichtung auf, die eingerichtet ist, um den Sichtschutz in die Ruhestellung vorzuspannen. Die Vorspanneinrichtung ist bevorzugt als in dem Distalteil angeordnete Schraubenfeder ausgebildet, welche mit dem Sichtschutz zusammenwirkt und diesen in die Ruhestellung drängt.

Der Sichtschutz wird bevorzugt in die Expositionsstellung verlagert, wenn die Injektionseinrichtung zum Durchführen einer Injektion auf den Körper eines Patienten aufgesetzt und gegen den Körper des Patienten gedrängt wird. Während der Sichtschutz in die Expositionsstellung verlagert wird, kann zugleich die Injektionsnadel in den Körper des Patienten eindringen. Dabei bleibt die Injektionsnadel zu jedem Zeitpunkt dem Blick des Anwenders verborgen, da stets der nicht in den Körper des Patienten eingedrungene Teil der Injektionsnadel noch von dem Sichtschutz verdeckt ist.

Bei der Ausgestaltung der Injektionseinrichtung, bei welcher der Sichtschutz verlagerbar an dem Distalteil gehalten ist, ist es insbesondere möglich, dass das Distalteil unlösbar mit dem Proximalteil verbunden ist, wie dies oben in Zusammenhang mit dem nicht zur Erfindung gehörenden Beispiel erläutert ist. Es ist aber auch möglich, dass das Distalteil - insbesondere zu Reparatur- oder Wartungszwecken - von dem Proximalteil lösbar ist, wie dies ebenfalls oben erläutert ist.

Gemäß einer Weiterbildung der Erfindung ist vorgesehen, dass eine distale Stirnseite des Distalteils die Ladeöffnung aufweist, durch die das Injektionsladeteil in das Distalteil eingebracht werden kann. In diesem Fall wird das Injektionsladeteil von der bestimmungsgemäß einer Injektionsstelle zugewandten Seite in das Distalteil eingeführt. Die Injektionseinrichtung ist insoweit quasi als Vorderlader ausgebildet. Insbesondere wird das Injektionsladeteil bevorzugt durch den Sichtschutz hindurch in das Distalteil eingebracht.

Bevorzugt ist das Injektionsladeteil in das Distalteil eindrehbar, insbesondere einschraubbar. Hierzu weisen bevorzugt das Distalteil einerseits und das Injektionsladeteil andererseits jeweils komplementäre Schraubmittel, auf. Besonders bevorzugt ist in dem Distalteil mindestens ein Gewindegang ausgebildet, insbesondere ein Rechtsgewinde, wobei vorzugsweise in besonders einfacher Ausgestaltung an dem Injektionsladeteil ein Einschraubvorsprung angeordnet ist, der eingerichtet ist, um mit dem Gewindegang formschlüssig zusammenzuwirken und in den Gewindegang eingeschraubt zu werden. Es bedarf dann keiner separaten Ausbildung eines Gewindes an dem Injektionsladeteil, sodass dieses besonders einfach ausgebildet sein kann. Vorzugsweise sind zwei solcher Gewindegänge in dem Distalteil angeordnet, wobei zugleich entsprechend das Injektionsladeteil zwei Einschraubvorsprünge aufweist.

Alternativ oder zusätzlich ist das Injektionsladeteil bevorzugt axial fixiert in dem Distalteil anordenbar. Es kann so eine sichere und stabile axiale Position für das Injektionsladeteil in dem Distalteil bereitgestellt werden, wobei das Injektionsladeteil axial fest relativ zu dem Distalteil in diesem gehalten ist. Insbesondere kann der wenigstens eine Gewindegang einen Gewindeauslauf aufweisen, durch den die Axialposition des Injektionsladeteils festgelegt wird. Alternativ ist es möglich, dass das Distalteil und das Injektionsladeteil einen Fixiermechanismus aufweisen, beispielsweise nach Art eines Bajonettverschluss.

Gemäß der Erfindung ist vorgesehen, dass das Distalteil einen Sichtschutz-Verriegelungsmechanismus aufweist, der eingerichtet ist, um den Sichtschutz in einer Sperrstellung - gegen axiale Verlagerung, insbesondere gegen Verlagerung zwischen der Ruhestellung und der Expositionsstellung - zu sperren, vorzugsweise zu verriegeln, und in einer Freigabestellung die Verlagerung des Sichtschutzes freizugeben. Vorteilhaft kann durch den Sichtschutz-Verriegelungsmechanismus verhindert werden, dass der Sichtschutz versehentlich in die Expositionsstellung verlagert und damit die Injektionsnadel außerhalb der Durchführung einer Injektion freigelegt wird. Ein ungewollter Anblick der Injektionsnadel oder - schlimmer noch - eine ungewollte Stichverletzung durch die Injektionsnadel kann/können so besonders sicher vermieden werden.

Vorzugsweise ist der Sichtschutz-Verriegelungsmechanismus eingerichtet, um beim Einbringen, insbesondere beim Eindrehen oder Einschrauben, des Injektionsladeteils in das Distalteil entsperrt zu werden. Er ist dabei insbesondere eingerichtet, um durch das Einbringen, insbesondere Eindrehen oder Einschrauben, des Injektionsladeteils in das Distalteil entsperrt zu werden. Dabei ist unter "Entsperren" zu verstehen, dass die Verlagerung des Sichtschutzes von der Ruhestellung in die Expositionsstellung freigegeben wird. In einfacher Weise und ohne Zusatzaufwand für den Anwender kann demnach die entsprechende Verlagerung des Sichtschutzes dadurch freigegeben werden, dass das Injektionsladeteil bestimmungsgemäß in das Distalteil eingebracht wird.

Alternativ oder zusätzlich ist der Sichtschutz-Verriegelungsmechanismus bevorzugt eingerichtet, um durch die dem Antrieb zugeordnete Kolbenstange gesperrt zu werden. Somit kann der Sichtschutz in vorteilhafter Weise ohne weiteren, zusätzlichen Eingriff des Anwenders beim Durchführen einer Injektion oder nach dem Durchführen einer Injektion - automatisch - wieder gesperrt werden. Bevorzugt wird der Sichtschutz-Verriegelungsmechanismus durch axiale Verlagerung der Kolbenstange in distaler Richtung gesperrt, also beim Durchführen der Injektion. Auf diese Weise wird sehr sicher eine versehentliche Stichverletzung nach dem Durchführen der Injektion verhindert, wenn typischerweise die Aufmerksamkeit des Anwenders geringer ist als vor dem Durchführen der Injektion.

Gemäß der Erfindung ist vorgesehen, dass der Sichtschutz-Verriegelungsmechanismus eine Drehscheibe aufweist, die um die Längsachse des Distalteils zwischen der Sperrstellung und der Freigabestellung drehbar in dem Distalteil angeordnet ist, wobei die Drehscheibe wenigstens eine Durchgangsaussparung aufweist, wobei die Drehscheibe mit der wenigstens einen Durchgangsaussparung derart eingerichtet und angeordnet ist, dass ein Sperrvorsprung des Sichtschutzes in der Freigabestellung durch die Durchgangsaussparung hindurch verlagert werden kann, wobei der Sperrvorsprung in der Sperrstellung - insbesondere von der distalen Seite her - an der Drehscheibe anschlägt. Mittels der Drehscheibe wird so eine einfache und funktionssichere Verriegelung für den Sichtschutz bereitgestellt. Ist die Drehscheibe in der Sperrstellung angeordnet, ist die Durchgangsaussparung insbesondere von dem Sperrvorsprung derart weggedreht, dass dieser nicht durch die Durchgangsaussparung hindurch verlagert werden kann, und daher vielmehr an der Drehscheibe anschlägt, sodass der Sichtschutz nicht aus der Ruhestellung in die Expositionsstellung verlagert werden kann. In der Freigabestellung ist dagegen die Drehscheibe mit der wenigstens einen Durchgangsaussparung so verschwenkt, dass sie einen Verlagerungsweg für den Sperrvorsprung freigibt, sodass dieser durch die Durchgangsaussparung hindurchtreten kann. Der Sichtschutz kann dann aus der Ruhestellung in die Expositionsstellung verlagert werden, sodass im Ergebnis eine Injektion durchgeführt werden kann.

Die Durchgangsaussparung ist bevorzugt als randoffene Ausnehmung in der Drehscheibe ausgebildet, insbesondere als radialer Rücksprungsbereich an einem äußeren Umfang der Drehscheibe.

Der Sperrvorsprung ist bevorzugt rampenförmig - in distaler Richtung abfallend - ausgebildet und an einer Federzunge angeordnet oder Teil der Federzunge. Dies hat den Vorteil, dass der Sichtschutz auch dann aus der Expositionsstellung zurück in die Ruhestellung verlagert werden kann, wenn die Drehscheibe in der Sperrstellung angeordnet ist. Wird beispielsweise der Sichtschutz-Verriegelungsmechanismus - hier die Drehscheibe - durch die Kolbenstange bei deren axialer Verlagerung von der Freigabestellung in die Sperrstellung verlagert, geschieht dies typischerweise, während der Sichtschutz noch in der Expositionsstellung angeordnet ist. Insbesondere wenn anschließend die Injektionseinrichtung von der Haut eines Patienten entfernt wird, wird der Sichtschutz aus der Expositionsstellung zurück in die Ruhestellung gedrängt. Der Sperrvorsprung kann dann gegen eine Vorspannkraft der Federzunge, deren Teil er ist oder an der er angeordnet ist, radial auswärts ausschwenken und somit an der Drehscheibe, insbesondere an einer äußeren Umfangslinie der Drehscheibe, vorbeigleiten, insbesondere aufgrund seiner rampenförmigen Ausgestaltung. Umgekehrt kann aber - gerade aufgrund der rampenförmigen oder auch keilförmigen Ausgestaltung des Sperrvorsprungs - dieser in der Sperrstellung nicht an der Drehscheibe vorbeigleiten, wenn versucht wird, den Sichtschutz aus der Ruhestellung in die Expositionsstellung zu verlagern. Insbesondere ermöglicht also die rampenförmige Ausgestaltung des Sperrvorsprungs an der Federzunge, dass dieser in der Sperrstellung der Drehscheibe von der proximalen Seite der Drehscheibe her an dieser vorbei auf deren distale Seite gelangen kann, nicht aber in umgekehrter Richtung.

In dem Distalteil sind bevorzugt zumindest zwei Raststellungen für die Drehscheibe vorgesehen, wobei die Drehscheibe insbesondere in der Sperrstellung einerseits und in der Freigabestellung andererseits in jeweils eine der Raststellungen eingreift und so bezüglich ihrer Winkellage sicher gehalten wird.

Gemäß der Erfindung ist vorgesehen, dass die Drehscheibe wenigstens eine Mitnahmefläche aufweist, die eingerichtet ist, um mit einem Mitnehmer des Injektionsladeteils zusammenzuwirken, sodass die Drehscheibe von der Sperrstellung in die Freigabestellung gedreht wird, wenn das Injektionsladeteil in das Distalteil eingedreht, insbesondere eingeschraubt wird. Dies stellt eine einfache und funktionssichere Möglichkeit dar, die Drehscheibe beim Eindrehen des Injektionsladeteils durch das Injektionsladeteil zu betätigen.

Die wenigstens eine Mitnahmefläche ist bevorzugt an einem Mitnahmevorsprung ausgebildet, der sich an der Drehscheibe und ausgehend von einer Stirnfläche der Drehscheibe in axialer Richtung, insbesondere in distaler Richtung, erstreckt. Der Mitnehmer ist komplementär bevorzugt als Mitnehmervorsprung an dem Injektionsladeteil, insbesondere an der Funktionshülse, ausgebildet, wobei er sich insbesondere ausgehend von einer distalen Stirnseite des Injektionsladeteils in proximaler Richtung erstreckt.

Vorzugsweise bilden die Mitnahmefläche und der Mitnehmer zusammen eine Klauenkupplung aus.

Vorzugsweise weist die Drehscheibe zwei Mitnahmeflächen, insbesondere zwei Mitnahmevorsprünge, auf, die sich besonders bevorzugt diametral gegenüberliegen. Entsprechend weist bevorzugt das Injektionsladeteil zwei Mitnehmer auf, wobei jeweils einer der Mitnehmer mit jeweils einer Mitnahmefläche der Drehscheibe zusammenwirkt.

Alternativ oder zusätzlich weist die Drehscheibe bevorzugt wenigstens eine Ablauffläche auf, auf der eine Aktuierungsstruktur der Kolbenstange ablaufen kann, derart, dass die Drehscheibe von der Freigabestellung in die Sperrstellung gedreht wird, wenn die Kolbenstange in einer bestimmten Richtung, das heißt proximal oder distal - axial relativ zu der Drehscheibe verlagert wird. Auf diese Weise kann die Verlagerung der Drehscheibe von der Freigabestellung in die Sperrstellung in einfacher und funktionssicherer Weise durch die Kolbenstange aktuiert werden.

Die Aktuierungsstruktur ist bevorzugt als Aktorvorsprung oder Aktorpin ausgebildet, wobei sich der Aktorvorsprung oder Aktorpin in radialer Richtung ausgehend von einer äußeren Umfangsfläche der Kolbenstange nach radial außen erstreckt.

Vorzugsweise weist die Drehscheibe zwei solcher Ablaufflächen auf, wobei die Kolbenstange zwei Aktuierungsstrukturen aufweist, wobei jede Aktuierungsstruktur jeweils mit einer der Ablaufflächen zusammenwirkt. Die Ablaufflächen und entsprechend die Aktuierungsstrukturen sind bevorzugt jeweils einander diametral gegenüberliegend angeordnet.

Insbesondere ist die wenigstens eine Ablauffläche so ausgebildet, dass die Drehscheibe gedreht wird, wenn die Kolbenstange mit der Aktuierungsstruktur relativ zu der Drehscheibe in distaler Richtung axial verlagert wird, insbesondere dann, wenn die Aktuierungsstruktur in distaler Richtung entlang der Ablauffläche abläuft, insbesondere gleitet. Das heißt insbesondere, dass die Drehscheibe bevorzugt von der Freigabestellung in die Sperrstellung gedreht wird, wenn durch die Kolbenstange eine Injektion bewirkt und die pharmazeutische Substanz aus dem Injektionsladeteil ausgetrieben wird.

In Umfangsrichtung neben der wenigstens einen Ablauffläche ist bevorzugt ein Durchgangsfenster angeordnet, durch welches die Aktuierungsstruktur bei einer umgekehrten axialen Verlagerung der Kolbenstange hindurchtreten kann, ohne die Drehscheibe zu verlagern. Insbesondere kann die Aktuierungsstruktur durch das Durchgangsfenster hindurchtreten, wenn die Kolbenstange in proximaler Richtung axial relativ zu der Drehscheibe verlagert wird. Es wird somit eine Drehung der Drehscheibe bei entgegengesetzter axialer Verlagerung der Kolbenstange, insbesondere proximaler Verlagerung der Kolbenstange, vermieden. Somit werden insbesondere die Funktionen der Verriegelung einerseits und der Entriegelung andererseits mit Bezug auf die Drehscheibe getrennt: Die Drehscheibe wird beim Eindrehen des Injektionsladeteils entriegelt, das heißt von der Sperrstellung in die Freigabestellung gedreht; sie wird verriegelt, das heißt von der Freigabestellung in die Sperrstellung gedreht, wenn die Kolbenstange relativ zu der Drehscheibe in die bestimmte Richtung, insbesondere in die distale Richtung, axial verlagert wird.

Gemäß einer Weiterbildung der Erfindung ist vorgesehen, dass die Injektionseinrichtung, insbesondere das Distalteil, einen verstellbaren Anschlag für den verlagerbaren Sichtschutz aufweist. Der Sichtschutz schlägt bevorzugt in der Expositionsstellung an dem verstellbaren Anschlag an. Mittels des verstellbaren Anschlags kann vorteilhaft eine Eindringtiefe der Injektionsnadel in den Körper eines Patienten eingestellt, insbesondere vorgegeben werden. Ist dabei der verstellbare Anschlag so ausgebildet, dass der Sichtschutz in der Expositionsstellung an dem verstellbaren Anschlag anschlägt, kann direkt die Lage des Sichtschutzes relativ zu dem Distalteil während der Injektion verändert werden, wodurch offensichtlich die Eindringtiefe der Injektionsnadel beeinflusst wird.

Hier enden diejenigen Weiterbildungen und bevorzugten Ausgestaltungen, die bevorzugt in Kombination miteinander das Ausführungsbeispiel bilden.

Die Aufgabe wird auch gelöst, indem ein Injektionsladeteil gemäß Anspruch 10 geschaffen wird. Weitere Ausgestaltungen sind in den abhängigen Ansprüchen beschrieben.

Das Injektionsladeteil ist eingerichtet zur Verwendung in einer erfindungsgemäßen Injektionseinrichtung oder in einer Injektionseinrichtung nach dem zuvor beschriebenen Ausführungsbeispiel. Das Injektionsladeteil weist ein Packmittelaufnahmeteil auf, in dem ein Primärpackmittel gehalten ist, wobei in dem Primärpackmittel eine pharmazeutische Substanz angeordnet ist. Das Injektionsladeteil ist insbesondere eingerichtet, um in dem Distalteil der Injektionseinrichtung aufgenommen zu werden. In Zusammenhang mit dem Injektionsladeteil ergeben sich insbesondere die Vorteile, die bereits in Zusammenhang mit der Injektionseinrichtung erläutert wurden.

Das Injektionsladeteil ist bevorzugt so ausgestaltet, wie dies zuvor bereits in Zusammenhang mit der Injektionseinrichtung explizit oder implizit erläutert wurde.

Gemäß einer Weiterbildung der Erfindung ist vorgesehen, dass das Packmittelaufnahmeteil eine Funktionshülse und ein Sicherungsteil aufweist, wobei das Primärpackmittel in der Funktionshülse aufgenommen ist, und wobei das Primärpackmittel durch das Sicherungsteil in der Funktionshülse gesichert, vorzugsweise fixiert ist. Die Funktionshülse ist bevorzugt eingerichtet, um das Injektionsladeteil in dem Distalteil der Injektionseinrichtung zu führen und zu halten. Alternativ oder zusätzlich - insbesondere in Zusammenhang mit dem erfindungsgemäßen Ausführungsbeispiel -, ist die Funktionshülse eingerichtet, um den Sichtschutz-Verriegelungsmechanismus zu entriegeln. Alternativ oder zusätzlich ist die Funktionshülse bevorzugt eingerichtet, um ein Nadelschutzteil zum manuellen Abziehen freizugeben, wenn das Injektionsladeteil in dem Distalteil angeordnet ist oder wird.

Dass das Primärpackmittel in der Funktionshülse aufgenommen und gesichert ist, bedeutet insbesondere, dass das Primärpackmittel in der Funktionshülse gehalten ist. Dies wiederum bedeutet insbesondere, dass das Primärpackmittel aus der Funktionshülse nicht herausfallen oder ungewollt aus der Funktionshülse gelöst oder von der Funktionshülse getrennt werden kann.

Das Sicherungsteil ist bevorzugt als Klemmring, als Clipsteil oder als Clipsring ausgebildet, der mit der Funktionshülse verklemmt oder verclipst wird, wenn das Primärpackmittel in der Funktionshülse angeordnet ist, um das Primärpackmittel in der Funktionshülse zu sichern, insbesondere zu fixieren.

Gemäß einer Weiterbildung der Erfindung ist vorgesehen, dass das Injektionsladeteil ein Nadelschutzteil aufweist, das mit der Funktionshülse lösbar verbunden ist und eine Injektionsnadel des Primärpackmittels verbirgt, wenn das Nadelschutzteil mit der Funktionshülse verbunden ist. Dies hat den Vorteil, dass die Injektionsnadel auch dann verborgen ist, wenn das Injektionsladeteil nicht in dem Distalteil der Injektionseinrichtung angeordnet ist. Um eine Injektion durchzuführen, muss das Nadelschutzteil von der Funktionshülse gelöst werden, insbesondere muss es von der Funktionshülse abgezogen werden.

Dass das Nadelschutzteil mit der Funktionshülse lösbar verbunden ist, bedeutet insbesondere, dass es unmittelbar mit der Funktionshülse verbunden ist, und/oder dass es mittelbar über das Primärpackmittel mit der Funktionshülse verbunden ist. Es kann also auch unmittelbar mit dem Primärpackmittel verbunden sein. Da dieses aber wiederum seinerseits mit der Funktionshülse verbunden ist, ist dann auch das Nadelschutzteil jedenfalls mittelbar mit der Funktionshülse verbunden.

Dass das Nadelschutzteil die Injektionsnadel verbirgt, bedeutet insbesondere, dass es diese optisch und haptisch verbirgt, derart, dass ein Anwender die Injektionsnadel nicht zu Gesicht bekommt, wobei auch versehentliche Stichverletzungen durch Berühren der Injektionsnadel verhindert werden, da die Injektionsnadel optisch und haptisch nicht zugänglich ist, wenn das Nadelschutzteil mit der Funktionshülse verbunden ist.

Gemäß einer Weiterbildung der Erfindung ist vorgesehen, dass das Nadelschutzteil eine äußere Griffhülse und ein innen in der Griffhülse mitnehmbar angeordnetes, elastisches Schutzelement aufweist. Die Injektionsnadel ist dabei in dem elastischen Schutzelement aufgenommen, wenn das Nadelschutzteil mit der Funktionshülse verbunden ist. Das Schutzelement kann insbesondere aus pharmazeutischem Gummi oder einem ähnlichen Material gebildet sein oder daraus bestehen. An der äußeren Griffhülse kann ein Anwender das Nadelschutzteil greifen und von der Funktionshülse abziehen, wenn er eine Injektion durchführen möchte. Dabei ragt das Nadelschutzteil mit der äußeren Griffhülse bevorzugt aus dem Distalteil heraus, und steht insbesondere auch über den Sichtschutz über, wenn das Injektionsladeteil in dem Distalteil aufgenommen ist, sodass ein Anwender die äußere Griffhülse des Nadelschutzteils leicht ergreifen und das Nadelschutzteil von der Funktionshülse abziehen kann.

Dass das elastische Schutzelement in der Griffhülse mitnehmbar angeordnet ist, bedeutet insbesondere, dass das elastische Schutzelement von der Injektionsnadel abgezogen wird, wenn die äußere Griffhülse von der Funktionshülse abgezogen wird. Insbesondere wird also das elastische Schutzelement mit der Griffhülse mitgenommen.

Gemäß der Erfindung ist vorgesehen, dass das Injektionsladeteil wenigstens einen Mitnehmer aufweist, der eingerichtet ist, um mit einer Mitnahmefläche der Drehscheibe des Sichtschutz-Verriegelungsmechanismus zusammenzuwirken, derart, dass die Drehscheibe von ihrer Sperrstellung in die Freigabestellung gedreht wird, wenn das Injektionsladeteil in das Distalteil eingebracht wird. Der Mitnehmer ist bevorzugt als axialer Vorsprung an dem Injektionsladeteil, insbesondere an der Funktionshülse, ausgebildet, der sich in proximaler Richtung erstreckt. Bevorzugt weist das Injektionsladeteil zwei solcher Mitnehmer auf. Vorzugsweise sind diese zwei Mitnehmer einander diametral gegenüberliegend an dem Injektionsladeteil ausgebildet.

Gemäß einer Weiterbildung der Erfindung ist bevorzugt vorgesehen, dass das Nadelschutzteil an der Funktionshülse verriegelt ist, derart, dass das Nadelschutzteil von der Funktionshülse nur getrennt werden kann, wenn das Injektionsladeteil in dem Distalteil angeordnet ist. Das Nadelschutzteil kann dann vorteilhaft nicht versehentlich von der Funktionshülse getrennt werden, solange das Injektionsladeteil außerhalb des Distalteils angeordnet ist und somit bestimmungsgemäß auch keine Injektion durchgeführt werden soll. Insbesondere auf diese Weise können sehr effizient Stichverletzungen eines Anwenders vermieden werden.

Dass das Nadelschutzteil an der Funktionshülse verriegelt ist, schließt auch ein, dass das Nadelschutzteil in einer bevorzugten Ausgestaltung an der Funktionshülse geklemmt ist. Es bedarf also nicht zwingend eines Formschlusses zwischen dem Nadelschutzteil und der Funktionshülse. Gleichwohl kann ein solcher Formschluss vorgesehen sein.

Dass das Nadelschutzteil nur von der Funktionshülse getrennt werden kann, wenn das Injektionsladeteil in dem Distalteil angeordnet ist, bedeutet insbesondere, dass nur dann eine zerstörungsfreie Trennung des Nadelschutzteils von der Funktionshülse - insbesondere ohne Gewaltanwendung - möglich ist.

Bevorzugt weist die Funktionshülse wenigstens einen elastisch radial nach außen vorgespannten Federarm, vorzugsweise zwei elastisch radial nach außen vorgespannte Federarme, auf, wobei der wenigstens eine Federarm das Nadelschutzteil hält. Der wenigstens eine Federarm wird gegen seine Vorspannung radial nach innen gedrängt und gibt damit das Nadelschutzteil frei, wenn das Injektionsladeteil in dem Distalteil angeordnet wird. Insbesondere wird der Federarm dann durch eine innere Umfangsfläche des Distalteils radial nach innen gedrängt.

Der wenigstens eine Federarm ist bevorzugt als Klemmarm oder Riegelarm ausgebildet.

Vorzugsweise ist wenigstens eine Betätigungsnase an dem Federarm angeordnet. Diese Betätigungsnase wirkt dann mit dem Distalteil zusammen, sodass der Federarm gegen die Vorspannung radial nach innen gedrängt wird, wenn das Injektionsladeteil in dem Distalteil angeordnet wird.

Die Erfindung wird im Folgenden anhand der Zeichnung näher erläutert. Dabei zeigen:
- Figur 1: ein nicht zur Erfindung gehörendes Beispiel einer Injektionseinrichtung sowie ein nicht zur Erfindung gehörendes Beispiel eines Injektionsladeteils für die Injektionseinrichtung;
- Figur 2: eine Detail-Längsschnittdarstellung der Injektionseinrichtung mit dem Ladeteil gemäß Figur 1 in einer ersten Funktionsstellung;
- Figur 3: die Injektionseinrichtung und das Ladeteil gemäß Figur 1 in einer zweiten Funktionsstellung;
- Figur 4: die Injektionseinrichtung und das Ladeteil gemäß Figur 1 in einer dritten Funktionsstellung;
- Figur 5: ein erfindungsgemäßes Ausführungsbeispiel einer Injektionseinrichtung sowie ein erfindungsgemäßes Ausführungsbeispiel eines Injektionsladeteils;
- Figur 6: eine Detail-Längsschnittdarstellung der Injektionseinrichtung mit dem Injektionsladeteil gemäß Figur 5;
- Figur 7: eine Detail-Längsschnittdarstellung der Injektionseinrichtung mit dem Injektionsladeteil gemäß Figur 5 in einer ersten Funktionsstellung;
- Figur 8: die Injektionseinrichtung und das Injektionsladeteil gemäß Figur 5 in einer zweiten Funktionsstellung;
- Figur 9: eine weitere Detail-Längsschnittdarstellung der Injektionseinrichtung und des Injektionsladeteils gemäß Figur 5, und
- Figur 10: eine Detail-Querschnittdarstellung der Injektionseinrichtung und des Injektionsladeteils gemäß Figur 5.

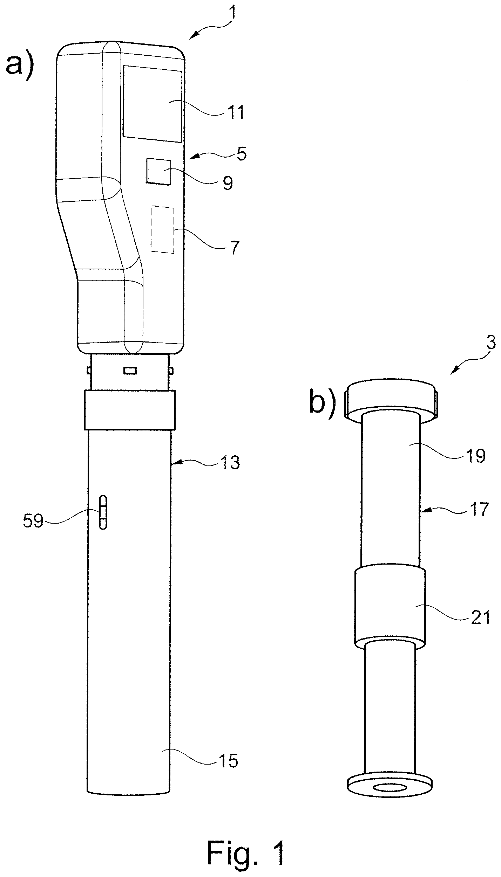

**Fig.** 1 zeigt bei a) ein nicht zur Erfindung gehörendes Beispiel einer Injektionseinrichtung 1 und bei b) ein nicht zur Erfindung gehörendes Beispiel eines Injektionsladeteils 3, das eingerichtet ist zur Verwendung mit der Injektionseinrichtung 1, insbesondere zur Verwendung in der Injektionseinrichtung 1.

Die Injektionseinrichtung 1 ist eingerichtet zum Injizieren einer pharmazeutischen Substanz und weist ein Proximalteil 5 auf, das auch als Backend bezeichnet wird. Das Proximalteil 5 weist insbesondere für den Betrachter nicht sichtbar, innenliegend und hier nur schematisch angedeutet, einen Antrieb 7 auf, der eingerichtet ist zum Bewirken der Injektion. Der Antrieb 7 kann beispielsweise einen Elektromotor aufweisen, der auf eine in Figur 1 nicht dargestellte Kolbenstange wirkt, um diese axial zu verlagern. Der Antrieb 7 kann energetisch aus einem Akkumulator oder einer Batterie gespeist sein. Es ist möglich, dass der Antrieb 7 über ein Getriebe mit der Kolbenstange wirkverbunden ist.

Die Injektionseinrichtung 1 weist außerdem bevorzugt ein Steuergerät auf, welches in Figur 1 ebenfalls nicht dargestellt und insbesondere innenliegend in dem Proximalteil 5 angeordnet ist. Das Steuergerät ist eingerichtet zur Ansteuerung des Antriebs 7; allgemein ist es bevorzugt eingerichtet zur Steuerung der Injektionseinrichtung 1 sowie zum Durchführen einer Injektion. Es ist möglich, dass das Steuergerät mit wenigstens einem Sensor verbunden ist, um die Injektion in Abhängigkeit von wenigstens einem durch den Sensor erfassten Parameter durchführen zu können.

Das Proximalteil 5 weist einen Betätigungstaster 9 auf, der insbesondere mit dem Steuergerät wirkverbunden und bevorzugt derart eingerichtet ist, dass durch Betätigung des Betätigungstasters 9 die Injektion gestartet und/oder beendet werden kann.

Weiterhin weist das Proximalteil 5 bevorzugt eine Anzeigeeinrichtung 11, insbesondere ein Display auf, das vorzugsweise ebenfalls mit dem Steuergerät verbunden und eingerichtet ist, um Anweisungen an den Anwender, insbesondere eine Schritt-für-Schritt-Anleitung, Parameterwerte, und/oder den Injektionsverlauf darzustellen.

Es ist möglich, dass das Proximalteil 5 als konventionelles Backend eines herkömmlichen Injektionspens ausgebildet ist.

Die Injektionseinrichtung 1 weist außerdem ein auch als Frontend bezeichnetes Distalteil 13 auf, das eingerichtet ist zur Aufnahme des Injektionsladeteils 3. Das Distalteil 13 ist dabei so ausgebildet, dass das Injektionsladeteil 3 axial in das Distalteil 13 eingebracht werden kann.

Eine Axialrichtung ist hier insbesondere eine Richtung der längsten Erstreckung der Injektionseinrichtung 1 und bevorzugt auch des Distalteils 13, insbesondere eine sich in Figur 1a) vertikal erstreckende Richtung. Eine Umfangsrichtung umgreift die Axialrichtung konzentrisch. Eine Radialrichtung steht senkrecht auf der Axialrichtung.

Die Injektionseinrichtung 1 weist außerdem einen Sichtschutz 15 auf, der so ausgebildet und angeordnet ist, dass eine in Figur 1 nicht dargestellte Injektionsnadel des Injektionsladeteils 3 während einer Anwendung der Injektionseinrichtung 1 für den Anwender verborgen ist. Hierdurch gestaltet sich die Injektion insbesondere psychologisch angenehmer. Zugleich ist bevorzugt ein Stichschutz durch den Sichtschutz 15 bereitgestellt, der verhindert, dass der Anwender sich versehentlich an der Injektionsnadel verletzt.

Der Begriff "distal" bezieht sich hierbei auf eine bestimmungsgemäß einer Injektionsstelle, insbesondere auf der Haut eines Patienten, zugewandte Richtung an der Injektionseinrichtung 1, das heißt in Figur 1a) insbesondere auf das untere Ende; der Begriff "proximal" kennzeichnet demgegenüber eine der Injektionsstelle abgewandte Richtung an der Injektionseinrichtung 1, in Figur 1a) demnach insbesondere das obere Ende der Injektionseinrichtung 1.

Das Distalteil 13 ist bei dem nicht zur Erfindung gehörenden Beispiel lösbar mit dem Proximalteil 5 verbunden. Hierzu ist insbesondere ein Verbindungsmechanismus vorgesehen, der beispielsweise nach Art eines Bajonettverschlusses ausgestaltet sein kann.

Die Injektionseinrichtung 1 weist den Antrieb 7 bevorzugt als einzigen Antrieb auf. Die Injektionseinrichtung 1 ist bevorzugt so eingerichtet, dass wenigstens eine andere mechanische Funktion der Injektionseinrichtung durch die Verlagerung der Kolbenstange bewirkt wird, insbesondere ein Einstechen der Injektionsnadel unter dem Sichtschutz 15, und/oder ein Verriegeln und/oder ein Entriegeln des Injektionsladeteils 3 und/oder des Sichtschutzes 15.

Die Injektionseinrichtung 1 ist bevorzugt als Pen oder Autoinjektor ausgebildet.

Das Injektionsladeteil 3 weist ein Packmittelaufnahmeteil 17 auf, in dem ein in Figur 1b) verdecktes Primärpackmittel gehalten ist. In dem Primärpackmittel ist die pharmazeutische Substanz für die Injektion angeordnet. Die pharmazeutische Substanz ist aus dem Injektionsladeteil 3 heraus injizierbar, wenn das Injektionsladeteil 3 in dem Distalteil 13 angeordnet ist.

Das Packmittelaufnahmeteil 17 weist bevorzugt eine Funktionshülse 19 auf, in der das Primärpackmittel aufgenommen ist. Außerdem weist das Packmittelaufnahmeteil 17 ein in Figur 1 nicht dargestelltes Sicherungsteil auf, durch das das Primärpackmittel in der Funktionshülse 19 gesichert, insbesondere fixiert ist.

Das Injektionsladeteil 3 weist ein Nadelschutzteil 21 auf, das mit der Funktionshülse 19 lösbar verbunden ist und die Injektionsnadel des Primärpackmittels verbirgt, wenn das Nadelschutzteil 21 mit der Funktionshülse 19 verbunden ist.

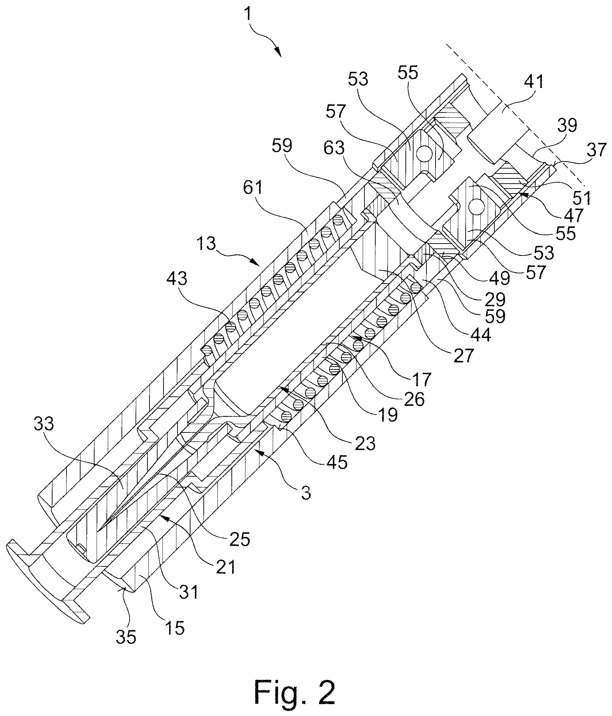

Fig. 2 zeigt eine Detail-Längsschnittdarstellung des nicht zur Erfindung gehörenden Beispiels der Injektionseinrichtung 1 mit dem nicht zur Erfindung gehörenden Beispiel des Injektionsladeteils 3 gemäß Figur 1, wobei das Injektionsladeteil 3 in dem Distalteil 13 aufgenommen ist.

Gleiche und funktionsgleiche Elemente sind in allen Figuren mit denselben Bezugszeichen bezeichnet, sodass insoweit jeweils auf die vorangegangene Beschreibung verwiesen wird.

In der Schnittdarstellung von Figur 2 ist ein in der Funktionshülse 19 aufgenommenes Primärpackmittel 23 erkennbar, das insbesondere als Spritze oder Karpule ausgebildet ist. Das Primärpackmittel 23 weist eine Injektionsnadel 25 auf, die hier fest mit einem Packmittelkörper 26 des Primärpackmittels 23 verbunden, insbesondere mit einem distalen Ende des Packmittelkörpers 26 verklebt ist. In dem Primärpackmittel 23 ist eine nicht dargestellte pharmazeutische Substanz angeordnet, außerdem ein innerhalb des Primärpackmittels 23 in axialer Richtung verlagerbarer Stopfen oder Kolben 27, durch dessen Verlagerung in für sich genommen bekannter Weise die pharmazeutische Substanz aus dem Primärpackmittel 23 ausgetrieben werden kann.

Mit der Funktionshülse 19 ist hier ein Sicherungsteil 29 verbunden, durch welches das Primärpackmittel 23 sicher und stabil in der Funktionshülse 19 gehalten wird. Das Sicherungsteil 29 ist bevorzugt als Klemmring, als Clipsteil oder als Clipsring ausgebildet.

Das Nadelschutzteil 21 weist eine äußere Griffhülse 31 und ein innen in der Griffhülse 31 mitnehmbar angeordnetes, elastisches Schutzelement 33, vorzugsweise aus pharmazeutischem Gummi, auf. Die Injektionsnadel 25 ist in dem elastischen Schutzelement 33 aufgenommen, wenn das Nadelschutzteil 21 mit der Funktionshülse 19 verbunden ist. An der äußeren Griffhülse 31 kann der Anwender das Nadelschutzteil 21 ergreifen und von der Funktionshülse 19 abziehen. Dabei wird zugleich das elastische Schutzelement 33 mitgenommen und von der Injektionsnadel 25 abgezogen. Diese wird hierdurch freigelegt, sodass eine Injektion möglich ist. Die Griffhülse 31 steht insbesondere in distaler Richtung über eine distale Stirnseite 35 des Distalteils 13 vor, sodass sie einfach von dem Anwender ergriffen und abgezogen werden kann. Auch nach dem Abziehen der Griffhülse 31 und des elastischen Schutzelements 33 ist die Injektionsnadel 25 jedenfalls zunächst noch innerhalb des Sichtschutzes 15 angeordnet und somit optisch sowie bevorzugt auch haptisch vor dem Anwender verborgen.

Bei dem nicht zur Erfindung gehörenden Beispiel ist der Sichtschutz 15 starr an dem Distalteil 13 vorgesehen. Insbesondere ist der Sichtschutz 15 durch das Distalteil 13 gebildet. Der Sichtschutz 15 ist hier insbesondere ein Teil einer Umfangswandung 61 des Distalteils 13.

Eine proximale Stirnseite 37 des Distalteils 13 weist eine Ladeöffnung 39 auf, durch die das Injektionsladeteil 3 in das Distalteil 13 eingebracht werden kann. Hierfür kann der Anwender zunächst das Distalteil 13 von dem Proximalteil 5 lösen und anschließend das Injektionsladeteil 3 in das Distalteil 13 einbringen. Danach kann das Distalteil 13 wieder mit dem Proximalteil 5 verbunden werden.

Wie in Figur 2 erkennbar, ragt in mit dem Proximalteil 5 verbundenem Zustand auch eine Kolbenstange 41 des Proximalteils 5, die durch den Antrieb 7 in Axialrichtung verlagerbar ist, durch die Ladeöffnung 39 in das Distalteil 13 hinein.

In dem Distalteil 13 ist bevorzugt eine Federeinrichtung 43 angeordnet, die hier als Schraubenfeder ausgebildet ist. Das Injektionsladeteil 3 stützt sich mit einem Flansch 44 an einem proximalen Ende der Federeinrichtung 43 ab. Die Federeinrichtung 43 stützt sich ihrerseits mit ihrem distalen Ende an einer Schulter 45 des Distalteils 13 ab. Das Injektionsladeteil 3 ist in Figur 2 in einer Schutzstellung dargestellt. In der Schutzstellung ist die Injektionsnadel 25 vollständig innerhalb des Distalteils 13 und damit insbesondere innerhalb des Sichtschutzes 15 angeordnet. Das Injektionsladeteil 3 kann innerhalb des Distalteils 13 entgegen der Federkraft der Federeinrichtung 43 in distaler Richtung in eine Injektionsstellung verlagert werden, wobei die Injektionsnadel 25 in der Injektionsstellung teilweise aus dem Distalteil 13 herausragt, insbesondere über die distale Stirnseite 35 vorsteht. In dieser Injektionsstellung ist eine Injektion durchführbar. Wie im Folgenden noch erläutert wird, wird die Verlagerung des Injektionsladeteils 3 in dem Distalteil 13 durch die Kolbenstange 41 bewirkt.

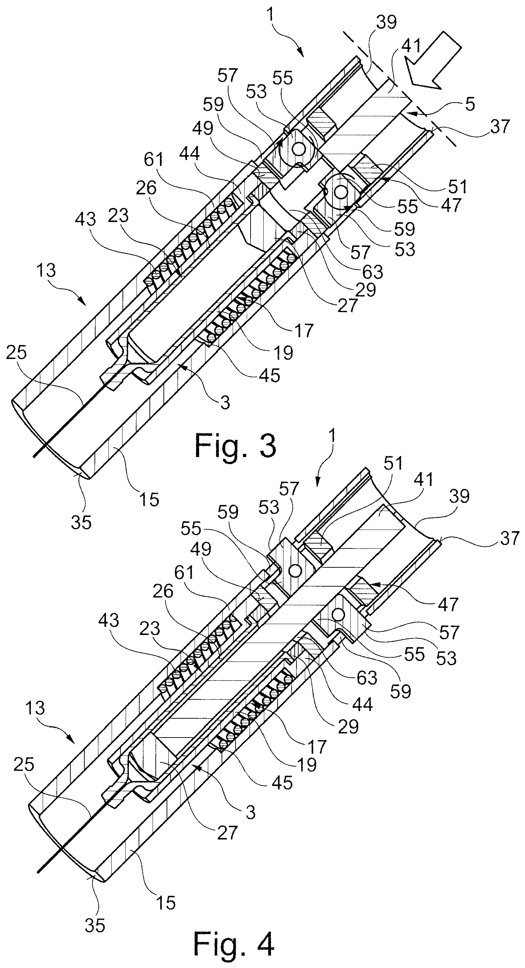

Die Injektionseinrichtung 1 weist ein Verriegelungsteil 47 auf, das durch die Ladeöffnung 39 in das Distalteil 13 eingeführt werden kann. Das Verriegelungsteil 47 ist eingerichtet, um an einer proximalen Anlagefläche 49 des Injektionsladeteils 3 anzuliegen. Das Verriegelungsteil 47 weist einen Grundkörper 51 und wenigstens ein an dem Grundkörper 51 schwenkbar angelenktes Riegelelement 53, bei dem hier dargestellten nicht zur Erfindung gehörenden Beispiel zwei einander diametral gegenüberliegend schwenkbar an dem Grundkörper 51 angelenkete Riegelelement 53, auf. Die Funktionsweise des Verriegelungsteils 47 in Zusammenhang mit der Durchführung einer Injektion ist im Folgenden anhand der Figuren 3 und 4 näher erläutert.

Das Verriegelungsteil 47 kann an dem Proximalteil 5 mitnehmbar und zugleich relativ zu dem Proximalteil 5 verlagerbar befestigt sein, derart, dass zugleich die Kolbenstange 41 relativ zu dem Verriegelungsteil 47 verlagerbar ist. Diese Ausgestaltung hat den Vorteil, dass das Verriegelungsteil 47 einfach zu handhaben ist und nicht verlorengehen kann. Es ist aber alternativ auch möglich, dass das Verriegelungsteil 47 als separates Element ausgebildet ist, das insbesondere nach dem Einbringen des Injektionsladeteils 3 in das Distalteil 13 durch die Ladeöffnung 39 eingeführt wird. Es kann dann wieder aus dem Distalteil 13 über die Ladeöffnung 39 entnommen werden, um nach Durchführung der Injektion das Injektionsladeteil 3 zu entnehmen.

Die Riegelelemente 53 sind L-förmig ausgebildet, mit einem Innenschenkel 55 und einem Außenschenkel 57, wobei die Riegelelemente 53 jeweils im Bereich einer Verbindung oder eines Schnittpunkts zwischen dem Innenschenkel 55 und dem Außenschenkel 57 schwenkbar an dem Grundkörper 51 angelenkt sind. Vorzugsweise stehen der Innenschenkel 55 und der Außenschenkel 57 senkrecht aufeinander.

Das Distalteil 13 weist wenigstens eine Riegelöffnung 59, hier insbesondere zwei einander diametral gegenüberliegende Riegelöffnungen 59 auf, die insbesondere als Fenster in der Umfangswandung 61 des Distalteils 13 ausgebildet sind. Eine der Riegelöffnungen 59 ist auch in Figur 1a) dargestellt.

In Figur 2 ist das Verriegelungsteil 47 in einer ersten Axialposition innerhalb des Distalteils 13 dargestellt, die auch als Entriegelungsstellung bezeichnet wird, wobei das Verriegelungsteil 47 - in axialer Richtung - entfernt von den Riegelöffnungen 59 angeordnet ist. In dieser ersten Axialposition und Entriegelungsstellung sind die Riegelelemente 53 in den Grundkörper 51 eingeschwenkt.

Das Verriegelungsteil 47 weist eine axiale Durchgriffsöffnung 63 für die Kolbenstange 41 auf. Die Durchgriffsöffnung 63 durchsetzt den Grundkörper 51 derart, dass die Kolbenstange 41 den Grundkörper 51 durch die Durchgriffsöffnung 63 zumindest in einer Funktionsstellung des Verriegelungsteils 47 durchgreifen kann.

In der in der Figur 2 dargestellten ersten Axialposition, das heißt der Entriegelungsstellung, greifen die Riegelelemente 53 - hier mit den Innenschenkeln 55 - in die Durchgriffsöffnung 63 ein, sodass diese für die Kolbenstange 41 gesperrt ist.

Das Verriegelungsteil 47 ist daher - gemeinsam mit dem Injektionsladeteil 3 - durch die Kolbenstange 41 verlagerbar und wird durch diese mitgenommen, wenn die Kolbenstange 41 in distaler Richtung verlagert wird. Dabei wird zugleich auch das Primärpackmittel 23 mit der Injektionsnadel 25 in distaler Richtung verlagert.

**Fig.** 3 zeigt das nicht zur Erfindung gehörende Beispiel der Injektionseinrichtung 1 mit dem Injektionsladeteil 3 bei abgezogenem Nadelschutzteil 21 und in einer Funktionsstellung, bei welcher das Verriegelungsteil 47 durch die Kolbenstange 41 aus der ersten Axialposition heraus in Richtung einer zweiten Axialposition verlagert wurde, wobei diese zweite Axialposition auch als Verriegelungsstellung bezeichnet wird. Allerdings ist in Figur 3 das Verriegelungsteil 47 noch nicht in der zweiten Axialposition angekommen. Es ist aber erkennbar, dass die Injektionsnadel 25 bereits über den Sichtschutz 15 und insbesondere die distale Stirnseite 35 hinaussteht, und dass die Federeinrichtung 43 im Vergleich zu Figur 2 komprimiert ist. Bei der axialen Verlagerung des Verriegelungsteils 47 gelangen die Riegelelemente 53 in den Bereich der Riegelöffnungen 59. Wie in Figur 3 dargestellt, können sie in diesem Bereich radial nach außen ausschwenken.

Fig. 4 zeigt das nicht zur Erfindung gehörende Beispiel der Injektionseinrichtung 3 mit dem Injektionsladeteil 3 in einer weiteren Funktionsstellung, hier nämlich nun in der zweiten Axialposition des Verriegelungsteils 47, das heißt in der Verriegelungsstellung, wobei zugleich nunmehr das Injektionsladeteil 3 in der Injektionsstellung angeordnet ist. In der zweiten Axialposition des Verriegelungsteils 47 schwenken die Riegelelemente 53 auf Höhe der Riegelöffnungen 59 aus und durchgreifen - insbesondere mit den Außenschenkeln 57 - die Riegelöffnungen 59. Zugleich geben die Riegelelemente 53 die Durchgriffsöffnung 63 für die Kolbenstange 41 frei, insbesondere indem die Innenschenkel 55 zumindest so weit aus der Durchgriffsöffnung 63 hinausschwenken, dass die Kolbenstange 41 zwischen den Innenschenkeln 55 hindurchgreifen kann. Somit kann auch die Kolbenstange 41 durch die Durchgriffsöffnung 63 hindurchgreifen und relativ zu dem Verriegelungsteil 47 verlagert werden. Die Kolbenstange 41 kann nun bei relativ zu dem Distalteil 13 festgehaltener Axialposition des Injektionsladeteils 3 den Kolben 27 innerhalb des Primärpackmittels 23 relativ zu diesem in distaler Richtung verlagern und damit die pharmazeutische Substanz aus dem Primärpackmittel 23 austreiben. Auf diese Weise wird in der Injektionsstellung des Injektionsladeteils 3 die Injektion durchgeführt.

Eine axiale Verlagerung des Injektionsladeteils 3 relativ zu dem Distalteil 13 ist währenddessen durch die die Riegelöffnungen 59 durchgreifenden Riegelelemente 53 gesperrt oder zumindest begrenzt, sowohl in distaler Richtung, soweit nicht bereits eine weitere distale Verlagerung des Injektionsladeteils 3 durch die auf Block gefahrene Federeinrichtung 43 begrenzt ist, als auch in proximaler Richtung, sodass bei durch das Gleiten des Kolbens 27 in dem Primärpackmittel 23 herabgesetzter Reibung das Injektionsladeteil 3 nicht innerhalb des Distalteils 13 unter der Federkraft der Federeinrichtung 43 zurückgezogen und damit eventuell aus der Haut des Patienten ausgefahren werden kann. Eine genaue Position der Riegelelemente 53 innerhalb der Riegelöffnungen 59 - in axialer Richtung - stellt sich dabei insbesondere abhängig von der Gleitreibung des Kolbens 27 in dem Primärpackmittel 23 einerseits sowie der Rückstellkraft der Federeinrichtung 43 andererseits während der Injektion ein.

Wird die Kolbenstange 41 insbesondere nach vollendeter Injektion in proximaler Richtung zurückgezogen, und gelangt sie dabei außer Eingriff mit den Riegelelementen 53, wird das Injektionsladeteil 3 zusammen mit dem Verriegelungsteil 47 durch die Kraft der Federeinrichtung 43 in die proximale Richtung gedrängt. Dabei schwenken nun die Riegelelemente 53 wieder in den Grundkörper 51 ein, und das Injektionsladeteil 3 wird durch die Federeinrichtung 43 zurück in seine Schutzstellung verlagert.

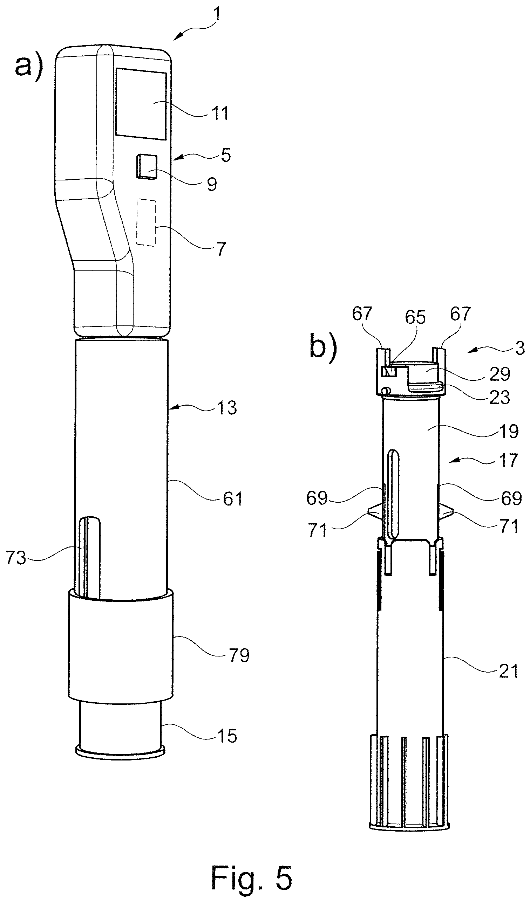

Fig. 5 zeigt bei a) ein erfindungsgemäßes Ausführungsbeispiel einer Injektionseinrichtung 1 und bei b) ein erfindungsgemäßes Ausführungsbeispiel eines Injektionsladeteils 3, welches eingerichtet ist, um mit der Injektionseinrichtung 1, insbesondere um in der Injektionseinrichtung 1 gemäß dem erfindungsgemäßen Ausführungsbeispiel verwendet zu werden.

Bei diesem erfindungsgemäßen Ausführungsbeispiel der Injektionseinrichtung 1 ist der Sichtschutz 15 verlagerbar an dem Distalteil 13 gehalten, insbesondere axial verlagerbar, insbesondere zwischen einer Ruhestellung und einer Expositionsstellung. Dabei ist der Sichtschutz 15 in Figur 5 in der Ruhestellung dargestellt.

Bevorzugt ist das Distalteil 13 bei diesem erfindungsgemäßen Ausführungsbeispiel der Injektionseinrichtung 1 unlösbar mit dem Proximalteil 5 verbunden. Es ist aber auch eine Ausgestaltung möglich, bei der das Distalteil 13 lösbar mit dem Proximalteil 5 verbunden ist, beispielsweise zu Wartungs- oder Reparaturzwecken.

Das Injektionsladeteil 3 weist - wie bei dem nicht zur Erfindung gehörenden Beispiel - auch gemäß dem erfindungsgemäßen Ausführungsbeispiel das Packmittelaufnahmeteil 17 mit der Funktionshülse 19 und dem Sicherungsteil 29 auf, welches hier als Clipsring ausgebildet ist und insbesondere mit Clipsvorsprüngen in Clipsaussparungen 65, von denen hier nur eine dem Betrachter zugewandt und daher dargestellt ist, der Funktionshülse 19 eingreift. In der Funktionshülse 19 des Packmittelaufnahmeteils 17 ist das Primärpackmittel 23 angeordnet und dort durch das Sicherungsteil 29 gesichert.

Das Injektionsladeteil 3 gemäß dem erfindungsgemäßen Ausführungsbeispiel weist wenigstens einen Mitnehmer 67, hier zwei einander diametral gegenüberliegende Mitnehmer 67, auf, deren Funktion im Folgenden noch erläutert wird. Die Mitnehmer 67 sind insbesondere als proximale Vorsprünge an der Funktionshülse 19 ausgebildet.

Bei dem erfindungsgemäßen Ausführungsbeispiel ist das Nadelschutzteil 21 bevorzugt an der Funktionshülse 19 verriegelt, derart, dass das Nadelschutzteil 21 nur zerstörungsfrei von der Funktionshülse 19 getrennt werden kann, wenn das Injektionsladeteil 3 in dem Distalteil 13 angeordnet ist. Dazu weist die Funktionshülse 19 hier insbesondere wenigstens eine, hier zwei radial nach außen elastisch vorgespannte Federarme 69 auf, die das Nadelschutzteil 21 verriegelt halten. An den Federarmen 69 ist jeweils eine Betätigungsnase 71 angeordnet. Wird das Injektionsladeteil 3 in das Distalteil 13 eingebracht, werden die Betätigungsnasen 71 durch die Umfangswandung 61 des Distalteils 13 radial nach innen gedrängt, wobei die Federarme 69 das Nadelschutzteil 21 freigeben, sodass dieses von der Funktionshülse 19 abgezogen werden kann.

Zurückkommend auf Figur 5a) ist in der Umfangswandung 61 des Distalteils 13 bevorzugt ein Sichtfenster 73 ausgebildet, durch welches der Anwender, insbesondere während der Durchführung einer Injektion, den Injektionsfortschritt und/oder einen Füllstand des Primärpackmittels 23 beobachten kann.

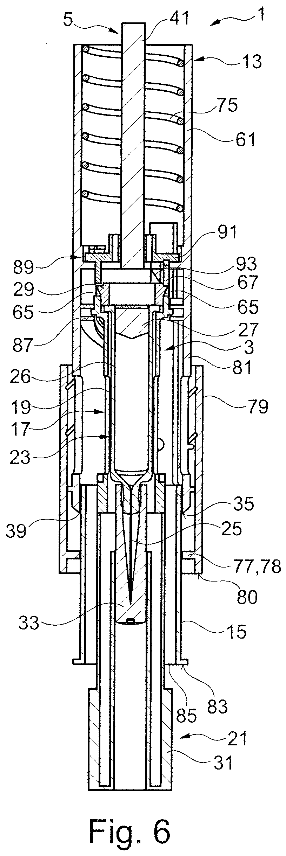

Fig. 6 zeigt eine Detail-Längsschnittdarstellung des erfindungsgemäßen Ausführungsbeispiels der Injektionseinrichtung 3 mit dem in dem Distalteil 13 aufgenommenen Injektionsladeteil 3.

Die Darstellung gemäß Figur 6 zeigt, dass der Sichtschutz 15 durch eine Vorspanneinrichtung 75 in die Ruhestellung vorgespannt ist. Die Vorspanneinrichtung 75 ist insbesondere innerhalb des Distalteils 13 angeordnet und bevorzugt als Schraubenfeder ausgebildet. Der Sichtschutz 15 ist axial und in proximaler Richtung gegen die Vorspannung der Vorspanneinrichtung 75 aus der Ruhestellung in die Expositionsstellung verlagerbar.

In Figur 6 ist noch dargestellt, dass die Injektionseinrichtung 1 bevorzugt einen verstellbaren Anschlag 77 für den verlagerbaren Sichtschutz 15 aufweist. Der Sichtschutz 15 schlägt dabei bevorzugt in der Expositionsstellung an dem verstellbaren Anschlag 77 an. Insbesondere trägt das Distalteil 13 an seinem äußeren Umfang eine Anschlaghülse 79, die den Anschlag 77 insbesondere als inneren Radialvorsprung, insbesondere als inneren Ringbund 78, oder als äußeren Ringbund oder einfach als distale Stirnfläche 80, aufweist, wobei der Sichtschutz 15 mit einem radial äußeren Ringbund in der Expositionsstellung an dem Anschlag 77 anschlägt. Der Anschlag 77 muss keine flächige Form aufweisen, es kann insbesondere auch wenigstens ein Anschlagpunkt vorgesehen sein. Eine Axialposition der Anschlaghülse 79 relativ zu einem Hülsenkörper 81 des Distalteils 13 ist dabei veränderbar, wobei zugleich die Axialposition des Anschlags 77 verändert wird. Vorzugsweise weisen hierfür die Anschlaghülse 79 einerseits und der Hülsenkörper 81 andererseits komplementäre, miteinander kämmende Gewindeteile auf, insbesondere der Hülsenkörper 81 ein Außengewinde und die Anschlaghülse 79 ein entsprechendes Innengewinde. Die Gewindeteile bilden bevorzugt miteinander ein selbsthemmendes Gewinde. Durch Verstellen der Anschlaghülse 79 wird somit insbesondere die Lage des Sichtschutzes 15 relativ zu dem Distalteil 13 in der Expositionsstellung während der Injektion und damit zugleich die Eindringtiefe der Injektionsnadel 25 in den Körper eines Patienten verändert.

Bei dem erfindungsgemäßen Ausführungsbeispiel der Injektionseinrichtung 1 weist die distale Stirnseite 35 des Distalteils 13 die Ladeöffnung 39 auf, durch die das Injektionsladeteil 3 in das Distalteil 13 eingebracht werden kann. Insbesondere weist eine distale Endfläche 83 des Sichtschutzes 15 eine entsprechende Sichtschutz-Ladeöffnung 85 auf, durch welche das Injektionsladeteil 3 in das Innere des Distalteils 13 eingebracht werden kann. Das Injektionsladeteil 3 wird also insbesondere durch den Sichtschutz 15 hindurch in das Distalteil 13 eingebracht. Dabei ist das Injektionsladeteil 3 bevorzugt in das Distalteil 13 eindrehbar, insbesondere einschraubbar. Hierzu weist das Distalteil 13 bevorzugt ein Schraubmittel, insbesondere wenigstens einen Gewindegang 87 auf, in den das Injektionsladeteil 3 bevorzugt mit wenigstens einem Einschraubvorsprung, vorzugsweise beidseitig mit zwei Einschraubvorsprüngen, eingreift, sodass das Injektionsladeteil 3 in den wenigstens einen Gewindegang 87 eingeschraubt werden kann.

Vorzugsweise ist das Injektionsladeteil 3 in dem Distalteil 13 axial fixiert anordenbar. Hierzu kann der wenigstens eine Gewindegang 87 insbesondere einen Gewindeauslauf aufweisen.

Das Distalteil 13 weist einen Sichtschutz-Verriegelungsmechanismus 89 auf, der eingerichtet ist, um den Sichtschutz 15 in einer Sperrstellung des Sichtschutz-Verriegelungsmechanismus 89 gegen axiale Verlagerung zwischen der Ruhestellung und der Expositionsstellung zu sperren, das heißt zu verriegeln, und in einer Freigabestellung diese Verlagerung freizugeben. Der Sichtschutz-Verriegelungsmechanismus 89 ist bevorzugt eingerichtet, um beim Einbringen, insbesondere Eindrehen oder Einschrauben, des Injektionsladeteils 3 in das Distalteil 13 entsperrt zu werden, und um durch die Kolbenstange 41, insbesondere während deren axialer Verlagerung, insbesondere in distaler Richtung, gesperrt zu werden. Hierzu weist der Sichtschutz-Verlagerungsmechanismus 89 bevorzugt eine Drehscheibe 91 auf, die um eine in Figur 6 vertikale Längsachse des Distalteils 13 zwischen der Sperrstellung und der Freigabestellung drehbar in dem Distalteil 13 angeordnet ist. In der Freigabestellung kann ein Sperrvorsprung 93 des Sichtschutzes 15 von der distalen Seite der Drehscheibe 91 auf deren proximale Seite verlagert werden, wobei der Sperrvorsprung 93 in der Sperrstellung auf der distalen Seite der Drehscheibe 91 an dieser anschlägt. Eine Verlagerung des Sichtschutzes 15 in die Expositionsstellung ist dann gesperrt.

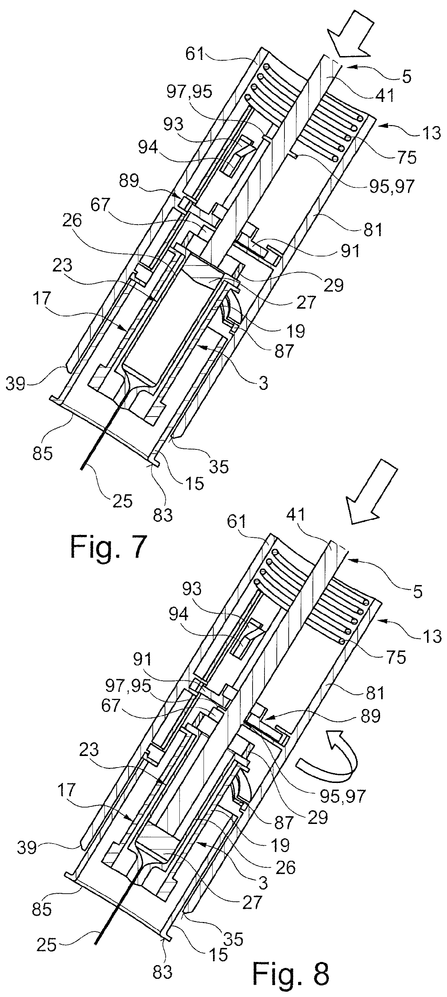

Fig. 7 zeigt eine weitere Längsschnitt-Detaildarstellung des erfindungsgemäßen Ausführungsbeispiels der Injektionseinrichtung 1 mit dem Injektionsladeteil 3 in einer ersten Funktionsstellung, hier nämlich der Freigabestellung des Sichtschutz-Verriegelungsmechanismus 89. Durch das Eindrehen oder Einschrauben des Injektionsladeteils 3 in das Distalteil 13 ist dabei die Drehscheibe 91 in die Freigabestellung verdreht worden, sodass der Sichtschutz 15 mit seinem Sperrvorsprung 93 in proximaler Richtung gegen die Vorspannkraft der Vorspanneinrichtung 75 in die Expositionsstellung verlagert ist. Im Vergleich zu Figur 6 ist nun also der Sperrvorsprung 93 nicht mehr auf der distalen Seite der Drehscheibe 91 angeordnet, sondern vielmehr auf deren proximaler Seite. Damit ist die Injektionsnadel 25 freigelegt, sodass durch distale Verlagerung der Kolbenstange 41 eine Injektion durchgeführt werden kann. Wohlgemerkt wird bei dem Ausführungsbeispiel das Injektionsladeteil 3 zum Zweck der Durchführung der Injektion nicht in axialer Richtung verlagert.

Fig. 8 zeigt eine der Darstellung von Figur 7 entsprechende Darstellung des erfindungsgemäßen Ausführungsbeispiels der Injektionseinrichtung 1 mit dem Injektionsladeteil 3 in einer zweiten Funktionsstellung. Dabei ist hier dargestellt, dass durch Verlagerung der Kolbenstange 41 in axialer Richtung zur Durchführung der Injektion eine Verdrehung der Drehscheibe 91 in die Sperrstellung durch die Kolbenstange 41 bewirkt wird. Die Kolbenstange 41 weist insoweit eine Aktuierungsstruktur 95, hier in Form von zwei Aktorpins 97 auf, wobei die Aktuierungsstruktur 95 die Drehscheibe 91 in Richtung ihrer Sperrstellung verdreht, wenn die Aktuierungsstruktur 95 die Drehscheibe von der proximalen Seite her kommend in Richtung der distalen Seite passiert.

Demgegenüber erfolgt keine weitere Verdrehung der Drehscheibe 91 bei der Zurückverlagerung der Kolbenstange 41 und insbesondere der Aktuierungsstruktur 95 von der distalen Seite auf die proximale Seite der Drehscheibe 91. Der Nadelschutz 15 kann nach Beenden der Injektion gleichwohl getrieben durch die Vorspannkraft der Vorspanneinrichtung 75 aus der Expositionsstellung in seine Ruhestellung zurückkehren, da der Sperrvorsprung 93 an einer Federzunge 94 angeordnet und rampenförmig ausgebildet ist, sodass er in Kontakt mit der Drehscheibe 91 nach radial außen ausweichen und so die Drehscheibe 91 aus proximaler Richtung her kommend zu der distalen Seite hin passieren kann.

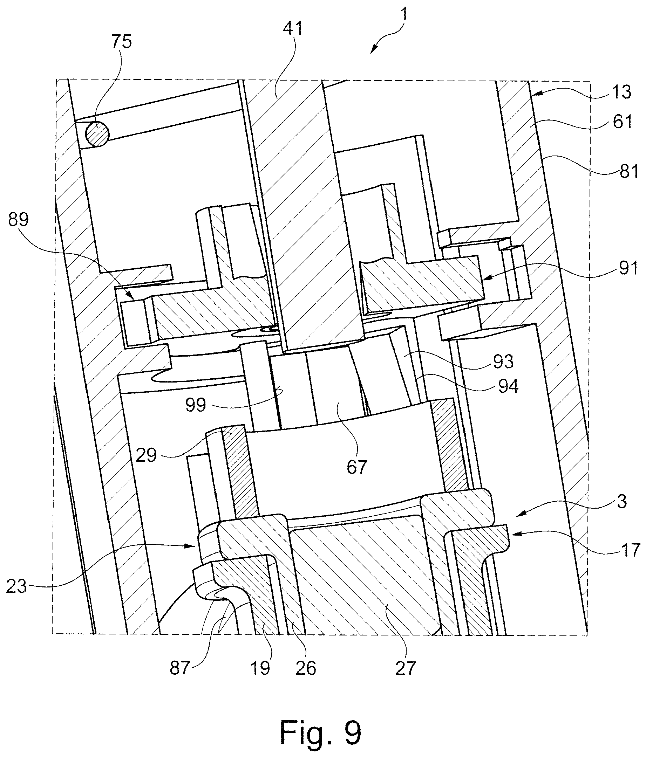

Fig. 9 zeigt eine weitere Detaildarstellung des erfindungsgemäßen Ausführungsbeispiels der Injektionseinrichtung 1 mit dem Injektionsladeteil 3. Anhand dieser Darstellung wird deutlich, dass die Drehscheibe 91 wenigstens eine Mitnahmefläche 99, bevorzugt zwei einander insbesondere diametral gegenüberliegende Mitnahmeflächen 99 aufweist, wobei die Mitnahmefläche 99 eingerichtet ist, um mit einem der Mitnehmer 67 des Injektionsladeteils 3 zusammenzuwirken, derart, dass die Drehscheibe 91 von der Sperrstellung in die Freigabestellung gedreht wird, wenn das Injektionsladeteil 3 in das Distalteil 13 eingedreht wird. Die Mitnahmefläche 99 ist insbesondere an einem Mitnahmevorsprung angeordnet, der sich an der Drehscheibe 91 in distaler Richtung erstreckt. Insbesondere wirken der Mitnehmer 67 und die Mitnahmefläche 99 bevorzugt nach Art einer Klauenkupplung zusammen.

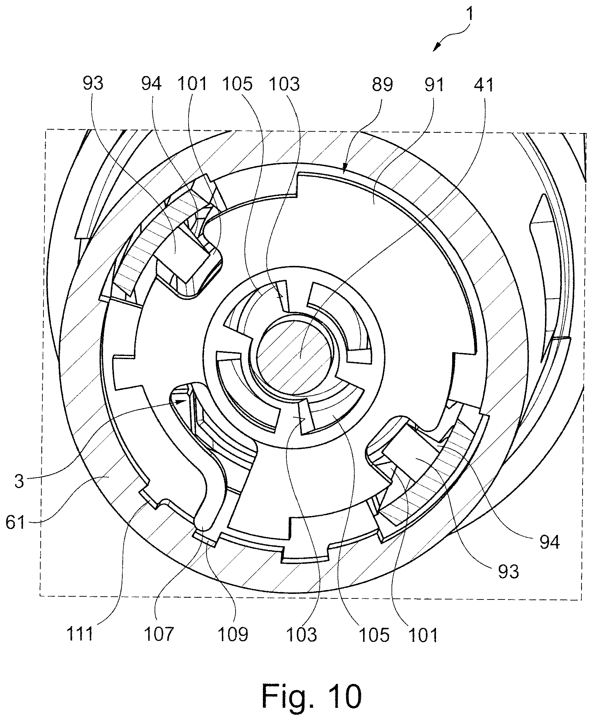

Fig. 10 zeigt eine Detail-Querschnittsdarstellung des erfindungsgemäßen Ausführungsbeispiels der Injektionseinrichtung 1 mit dem Injektionsladeteil 3, wobei die Querschnittsebene hier knapp oberhalb, das heißt proximal, der Drehscheibe 91 angeordnet ist.

In dieser Darstellung wird zum einen deutlich, dass die Drehscheibe 91 wenigstens eine Durchgangsaussparung 101, hier insbesondere zwei einander diametral gegenüberliegende Durchgangsaussparungen 101 aufweist, durch die in der Freigabestellung, die in Figur 10 dargestellt ist, der Sperrvorsprung 93 des Sichtschutzes 15 - hier zwei einander diametral gegenüberliegende Sperrvorsprünge 93 - hindurch verlagert werden können. Die Durchgangsaussparungen 101 sind insbesondere als radiale, randoffene Aussparungen ausgebildet.

Außerdem wird anhand der Darstellung von Figur 10 deutlich, dass die Drehscheibe 91 bevorzugt wenigstens eine Ablauffläche 103, hier insbesondere zwei, vorzugsweise einander diametral gegenüberliegende Ablaufflächen 103 aufweist, wobei auf den Ablaufflächen 103 die Aktuierungsstruktur 95, insbesondere die Aktorpins 97, der Kolbenstange 41 ablaufen können, wenn die Kolbenstange 41 in distaler Richtung verlagert wird. Dabei wird die Drehscheibe 91 von der Freigabestellung in die Sperrstellung gedreht, wenn die Kolbenstange 41 in distaler Richtung axial relativ zu der Drehscheibe 91 verlagert wird, insbesondere wenn die Aktuierungsstruktur 95, insbesondere die Aktorpins 97, die Drehscheibe von der proximalen Seite aus kommend in Richtung der distalen Seite passieren.

Dabei wird bevorzugt auch das Injektionsladeteil 3 in dem Gewindeauslauf um den entsprechenden Winkel zurückgedreht, wobei aber aufgrund der Ausgestaltung des Gewindeauslaufs des wenigstens einen Gewindegangs 87 bevorzugt keine oder höchstens eine geringfügige axiale Verlagerung des Injektionsladeteils 3 relativ zu dem Distalteil 13 bewirkt wird. Ein Mitdrehen der Injektionsnadel 25 im Körper des Patienten wird bevorzugt dadurch zumindest weitgehend vermieden, dass das Primärpackmittel 23 vorzugsweise frei drehbar in der Funktionshülse 19 aufgenommen ist.

In Umfangsrichtung neben den Ablaufflächen 103 sind jeweils Durchgangsfenster 105 ausgebildet, durch die die Aktuierungsstruktur 95 - vorzugsweise ohne Berührung der Drehscheibe 91 - von der distalen Seite zurück zu der proximalen Seite verlagert werden kann, wenn die Kolbenstange 41 zurückgezogen wird. Somit erfolgt keine Drehung der Drehscheibe 91 beim Zurückziehen der Kolbenstange 41 in proximaler Richtung nach dem Durchführen der Injektion.

In Figur 10 ist auch noch dargestellt, dass die Drehscheibe 91 bevorzugt eine Rastnase 107 aufweist, mit der sie in entsprechende Rastaussparungen 109, 111 in der Umfangswandung 61 des Distalteils 13 einerseits in der Freigabestellung und andererseits in der Sperrstellung einrasten kann. Dabei ist eine erste Rastaussparung 109 der in Figur 10 dargestellten Freigabestellung zugeordnet. Eine zweite Rastaussparung 111 ist der Sperrstellung zugeordnet. Insbesondere die Rastaussparung 109 sowie das Einrasten der Rastnase 107 darin dienen insbesondere auch einer mechanisch-haptischen - gegebenenfalls auch akustischen - Rückmeldung an den Anwender, dass das Injektionsladeteil 3 vollständig, das heißt injektionsbereit, in dem Distalteil 13 angeordnet ist.

Somit wird auch deutlich, dass in Blickrichtung des Betrachters von Figur 10 gesehen die Drehscheibe 13 beim Eindrehen des Injektionsladeteils 3 aus der Sperrstellung entgegen dem Uhrzeigersinn in die Freigabestellung gedreht wird, während sie dann, wenn die Aktuierungsstruktur 95 auf den Ablaufflächen 103 abläuft, im Uhrzeigersinn aus der Freigabestellung zurück in die Sperrstellung gedreht wird.

## Patentansprüche

1. Injektionseinrichtung (1) zum Injizieren einer pharmazeutischen Substanz, mit
- einem Proximalteil (5), das einen elektrischen Antrieb (7) zum Bewirken einer Injektion aufweist, und mit
- einem Distalteil (13), das eingerichtet ist zur Aufnahme eines Injektionsladeteils (3), das ein Packmittelaufnahmeteil (17) und ein in dem Packmittelaufnahmeteil (17) gehaltenes Primärpackmittel (23) aufweist, in dem eine pharmazeutische Substanz aufgenommen ist, wobei die pharmazeutische Substanz aus dem Injektionsladeteil (3) injizierbar ist, wenn das Injektionsladeteil (3) in dem Distalteil (13) angeordnet ist, wobei
- das Distalteil (13) so ausgebildet ist, dass das Injektionsladeteil (3) axial in das Distalteil (13) eingebracht werden kann, wobei
- die Injektionseinrichtung (1) einen Sichtschutz (15) aufweist, der so ausgebildet und angeordnet ist, dass eine Injektionsnadel (25) des Injektionsladeteils (3) während der Durchführung einer Injektion für einen Anwender verborgen ist, wobei
- der Sichtschutz (15) zusätzlich als Stichschutz ausgebildet und so angeordnet und ausgebildet ist, dass die Injektionsnadel (25) des Injektionsladeteils (3) während einer Anwendung der Injektionseinrichtung (1) abseits der vorzunehmenden Injektion für einen Anwender haptisch verborgen ist, wobei
- die Injektionseinrichtung (1) den elektrischen Antrieb (7) des Proximalteils (5) als einzigen Antrieb (7) aufweist, wobei
- das Distalteil (13) einen Sichtschutz-Verriegelungsmechanismus (89) aufweist, der eingerichtet ist, um den Sichtschutz (15) in einer Sperrstellung zu sperren und in einer Freigabestellung freizugeben, **dadurch gekennzeichnet, dass**
- der Sichtschutz-Verriegelungsmechanismus (89) eine Drehscheibe (91) aufweist, die um eine Längsachse des Distalteils (13) zwischen der Sperrstellung und der Freigabestellung drehbar in dem Distalteil (13) angeordnet ist, wobei die Drehscheibe (91) wenigstens eine Durchgangsaussparung (101) aufweist, durch die in der Freigabestellung ein Sperrvorsprung (93) des Sichtschutzes (15) hindurch verlagert werden kann, wobei der Sperrvorsprung (93) in der Sperrstellung an der Drehscheibe (91) anschlägt, wobei
- die Drehscheibe (91) wenigstens eine Mitnahmefläche (99) aufweist, die eingerichtet ist, um mit einem Mitnehmer (67) des Injektionsladeteils (3) zusammenzuwirken, so dass die Drehscheibe (91) von der Sperrstellung in die Freigabestellung gedreht wird, wenn das Injektionsladeteil (3) in das Distalteil (13) eingedreht wird.

2. Injektionseinrichtung (1) nach Anspruch 1, **dadurch gekennzeichnet, dass** der Antrieb (7) auf eine Kolbenstange (41) wirkt, um die Kolbenstange (41) axial zu verlagern, wobei die Injektionseinrichtung (1) vorzugsweise so eingerichtet ist, dass zusätzlich wenigstens eine andere mechanische Funktion der Injektionseinrichtung (1) durch die Verlagerung der Kolbenstange (41) bewirkt wird.

3. Injektionseinrichtung (1) nach einem der vorhergehenden Ansprüche, **dadurch gekennzeichnet, dass** die Injektionseinrichtung (1) als Pen oder als Autoinjektor ausgebildet ist.

4. Injektionseinrichtung (1) nach einem der vorhergehenden Ansprüche, **dadurch gekennzeichnet, dass** das Distalteil (13) lösbar mit dem Proximalteil (5) verbunden ist.

5. Injektionseinrichtung (1) nach einem der vorhergehenden Ansprüche, **dadurch gekennzeichnet, dass** der Sichtschutz (15) verlagerbar an dem Distalteil (13) gehalten ist.

6. Injektionseinrichtung (1) nach einem der vorhergehenden Ansprüche, **dadurch gekennzeichnet, dass** eine distale Stirnseite (35) des Distalteils (13) eine Ladeöffnung (39) aufweist, durch die das Injektionsladeteil (3) in das Distalteil (13) eingebracht werden kann, wobei das Injektionsladeteil (3) vorzugsweise in das Distalteil (13) eindrehbar, insbesondere einschraubbar ist, und/oder wobei das Injektionsladeteil (3) axial fixiert in dem Distalteil (13) anordenbar ist.

7. Injektionseinrichtung (1) nach einem der vorhergehenden Ansprüche, **dadurch gekennzeichnet, dass** der Sichtschutz-Verriegelungsmechanismus (89) eingerichtet ist, um beim Einbringen des Injektionsladeteils (3) in das Distalteil (13) entsperrt und durch eine dem Antrieb (7) zugeordnete Kolbenstange (41) gesperrt zu werden.

8. Injektionseinrichtung (1) nach einem der vorhergehenden Ansprüche, **dadurch gekennzeichnet, dass** die Drehscheibe (91)
a) wenigstens eine Ablauffläche (103) aufweist, auf der eine Aktuierungsstruktur (95) der Kolbenstange (41) ablaufen kann, derart, dass die Drehscheibe (91) von der Freigabestellung in die Sperrstellung gedreht wird, wenn die Kolbenstange (41) in eine bestimmte Richtung axial verlagert wird.

9. Injektionseinrichtung (1) nach einem der vorhergehenden Ansprüche, **dadurch gekennzeichnet, dass** die Injektionseinrichtung (1) einen verstellbaren Anschlag (77) für den verlagerbaren Sichtschutz (15) aufweist, wobei der Sichtschutz (15) vorzugsweise in einer Expositionsstellung, in der eine Injektion durchführbar ist, an dem verstellbaren Anschlag (77) anschlägt.

10. Injektionsladeteil (3) zur Verwendung in einer Injektionseinrichtung (1) nach einem der Ansprüche 1 bis 9, mit einem Packmittelaufnahmeteil (17), in dem ein Primärpackmittel (23) gehalten ist, wobei in dem Primärpackmittel (23) eine pharmazeutische Substanz angeordnet ist, **dadurch gekennzeichnet, dass** das Injektionsladeteil (3) wenigstens einen Mitnehmer (67) aufweist, der eingerichtet ist, um mit der wenigstens einen Mitnahmefläche (99) der Drehscheibe (91) des Distalteils (13) zusammenzuwirken, derart, dass die Drehscheibe (91) von der Sperrstellung in die Freigabestellung gedreht wird, wenn das Injektionsladeteil (3) in das Distalteil (13) eingedreht wird.

11. Injektionsladeteil (3) nach Anspruch 10, **dadurch gekennzeichnet, dass** das Packmittelaufnahmeteil (17) eine Funktionshülse (19) und ein Sicherungsteil (29) aufweist, wobei das Primärpackmittel (23) in der Funktionshülse (19) aufgenommen ist, und wobei das Primärpackmittel (23) durch das Sicherungsteil (29) in der Funktionshülse (19) gesichert ist.

12. Injektionsladeteil (3) nach einem der Ansprüche 10 oder 11, **dadurch gekennzeichnet, dass** das Injektionsladeteil (3) ein Nadelschutzteil (21) aufweist, das mit der Funktionshülse (19) lösbar verbunden ist und eine Injektionsnadel (25) des Primärpackmittels (23) verbirgt, wenn das Nadelschutzteil (21) mit der Funktionshülse (19) verbunden ist.

13. Injektionsladeteil (3) nach einem der Ansprüche 10 bis 12, **dadurch gekennzeichnet, dass** das Nadelschutzteil (21) eine äußere Griffhülse (31) und ein innen in der Griffhülse (31) mitnehmbar angeordnetes elastisches Schutzelement (33) aufweist, in dem die Injektionsnadel (25) aufgenommen ist, wenn das Nadelschutzteil (21) mit der Funktionshülse (19) verbunden ist.

14. Injektionsladeteil (3) nach einem der Ansprüche 10 bis 13, **dadurch gekennzeichnet, dass** das Nadelschutzteil (21) an der Funktionshülse (19) verriegelt ist, derart, dass das Nadelschutzteil (21) von der Funktionshülse (19) getrennt werden kann, wenn das Injektionsladeteil (3) in dem Distalteil (13) angeordnet ist.

## Claims

1. Injection device (1) for injecting a pharmaceutical substance, comprising
- a proximal part (5) that has an electric drive (7) for effecting an injection, and
- a distal part (13) that is configured to receive an injection loading part (3) that has a packaging receptacle part (17) and a primary packaging (23) held in the packaging receptacle part (17), in which a pharmaceutical substance is received, wherein the pharmaceutical substance is injectable from the injection loading part (3) when the injection loading part (3) is arranged in the distal part (13), wherein
- the distal part (13) is configured such that the injection loading part (3) can be inserted axially into the distal part (13), wherein
- the injection device (1) has a visual shield (15) that is configured and arranged such that an injection needle (25) of the injection loading part (3) is hidden from a user during execution of an injection, wherein
- the visual shield (15) is additionally configured as a puncture protection and is arranged and configured such that the injection needle (25) of the injection loading part (3) is haptically hidden from a user during use of the injection device (1) away from an injection to be executed, wherein
- the injection device (1) has the electric drive (7) of the proximal part (5) as a sole drive (7), wherein
- the distal part (13) has a visual-shield locking mechanism (89) that is configured to lock the visual shield (15) in a blocking position and to release the visual shield (15) in a release position, **characterized in that**
- the visual-shield locking mechanism (89) has a rotary disk (91) that is arranged rotatably in the distal part (13) about a longitudinal axis of the distal part (13) between the blocking position and the release position, wherein the rotary disk (91) has at least one passage recess (101) through which, in the release position, a blocking projection (93) of the visual shield (15) can be displaced, wherein the blocking projection (93) abuts against the rotary disk (91) in the blocking position, wherein
- the rotary disk (91) has at least one entrainment surface (99) that is configured to cooperate with an entrainer (67) of the injection loading part (3), such that the rotary disk (91) is rotated from the blocking position into the release position when the injection loading part (3) is rotated into the distal part (13).

2. Injection device (1) according to claim 1, **characterized in that** the drive (7) acts on a piston rod (41) to displace the piston rod (41) axially, wherein the injection device (1) is preferably configured such that additionally at least one other mechanical function of the injection device (1) is effected by the displacement of the piston rod (41).

3. Injection device (1) according to one of the preceding claims, **characterized in that** the injection device (1) is configured as a pen or as an autoinjector.

4. Injection device (1) according to one of the preceding claims, **characterized in that** the distal part (13) is detachably connected to the proximal part (5).

5. Injection device (1) according to one of the preceding claims, **characterized in that** the visual shield (15) is held displaceable on the distal part (13).

6. Injection device (1) according to one of the preceding claims, **characterized in that** a distal end face (35) of the distal part (13) has a loading opening (39) through which the injection loading part (3) can be inserted into the distal part (13), wherein the injection loading part (3) is preferably configured to be rotated into the distal part (13), in particular screwed in, and/or wherein the injection loading part (3) can be arranged axially fixed in the distal part (13).

7. Injection device (1) according to one of the preceding claims, **characterized in that** the visual-shield locking mechanism (89) is configured to be unlocked when the injection loading part (3) is inserted into the distal part (13) and to be locked by a piston rod (41) assigned to the drive (7).

8. Injection device (1) according to one of the preceding claims, **characterized in that** the rotary disk (91)
a) has at least one run-on surface (103) on which an actuation structure (95) of the piston rod (41) can run such that the rotary disk (91) is rotated from the release position into the blocking position when the piston rod (41) is displaced axially in a specific direction.

9. Injection device (1) according to one of the preceding claims, **characterized in that** the injection device (1) has an adjustable stop (77) for the displaceable visual shield (15), wherein the visual shield (15) preferably abuts against the adjustable stop (77) in an exposure position in which an injection can be executed.

10. Injection loading part (3) for use in an injection device (1) according to one of claims 1 to 9, with a packaging receptacle part (17) in which a primary packaging (23) is held, wherein a pharmaceutical substance is arranged in the primary packaging (23), **characterized in that** the injection loading part (3) has at least one entrainer (67) that is configured to cooperate with the at least one entrainment surface (99) of the rotary disk (91) of the distal part (13) such that the rotary disk (91) is rotated from the blocking position into the release position when the injection loading part (3) is rotated into the distal part (13).

11. Injection loading part (3) according to claim 10, **characterized in that** the packaging receptacle part (17) has a functional sleeve (19) and a securing part (29), wherein the primary packaging (23) is received in the functional sleeve (19), and wherein the primary packaging (23) is secured in the functional sleeve (19) by the securing part (29).

12. Injection loading part (3) according to one of claims 10 or 11, **characterized in that** the injection loading part (3) has a needle protection part (21) that is detachably connected to the functional sleeve (19) and that hides an injection needle (25) of the primary packaging (23) when the needle protection part (21) is connected to the functional sleeve (19).

13. Injection loading part (3) according to one of claims 10 to 12, **characterized in that** the needle protection part (21) has an outer grip sleeve (31) and an elastic protective element (33) that is arranged inside the grip sleeve (31) so as to be entrainable, in which the injection needle (25) is received when the needle protection part (21) is connected to the functional sleeve (19).

14. Injection loading part (3) according to one of claims 10 to 13, **characterized in that** the needle protection part (21) is locked to the functional sleeve (19) such that the needle protection part (21) can be separated from the functional sleeve (19) when the injection loading part (3) is arranged in the distal part (13).

## Revendications

1. Dispositif d'injection (1) pour injecter une substance pharmaceutique, avec
- une partie proximale (5) qui comporte un entraînement électrique (7) pour effectuer une injection, et avec
- une partie distale (13) qui est conçue pour accueillir une pièce de chargement d'injection (3) comportant une pièce de chargement d'emballage (17) et un emballage primaire (23) contenu dans la pièce de chargement d'emballage (17), dans lequel est accueillie une substance pharmaceutique, la substance pharmaceutique étant injectable à partir de la pièce de chargement d'injection (3) si la pièce de chargement d'injection (3) est située dans la partie distale (13), dans lequel
- la partie distale (13) est conçue de telle sorte que la pièce de chargement d'injection (3) puisse être introduite axialement dans la partie distale (13), dans lequel
- le dispositif d'injection (1) comporte un écran (15) qui est conçu et disposé de façon qu'une aiguille d'injection (25) de la pièce de chargement d'injection (3) soit cachée pendant la réalisation d'une injection pour un utilisateur, dans lequel
- l'écran (15) est en plus conçu comme une protection contre les piqûres et est situé et conçu de telle sorte que l'aiguille d'injection (25) de la pièce de chargement d'injection (3) ne puisse pas être touchée pendant une utilisation du dispositif d'injection (1) en dehors de l'injection à effectuer pour un utilisateur, dans lequel
- le dispositif d'injection (1) comporte l'entraînement électrique (7) de la partie proximale (5) comme unique entraînement (7), dans lequel
- la partie distale (13) comporte un mécanisme de blocage de l'écran (89) qui est conçu pour bloquer l'écran (15) dans une position de blocage et pour le débloquer dans une position de déblocage, **caractérisé en ce que**
- le mécanisme de blocage de l'écran (89) comporte un disque tournant (91) disposé de façon rotative dans la partie distale (13) autour d'un axe longitudinal de la partie distale (13) entre la position de blocage et la position de déblocage, dans lequel le disque tournant (91) comporte au moins un orifice traversant (101) à travers lequel une proéminence de blocage (93) de l'écran (15) peut venir se placer, la proéminence de blocage (93) venant s'accrocher dans la position de blocage sur le disque tournant (91), dans lequel
- le disque tournant (91) comporte au moins une surface de verrouillage (99) qui est conçue pour coopérer avec un cliquet de verrouillage (67) de la pièce de chargement d'injection (3), de façon que le disque tournant (91) soit tourné de la position de blocage en position de déblocage quand la pièce de chargement d'injection (3) est introduite par rotation dans la partie distale (13).

2. Dispositif d'injection (1) selon la revendication 1, **caractérisé en ce que** l'entraînement (7) agit sur une tige de piston (41) pour déplacer la tige de piston (41) axialement, le dispositif d'injection (1) étant préférentiellement conçu de sorte qu'en plus, au moins une autre fonction mécanique du dispositif d'injection (1) est effectuée par le déplacement de la tige de piston (41).

3. Dispositif d'injection (1) selon l'une des revendications précédentes, **caractérisé en ce que** le dispositif d'injection (1) est conçu comme un stylo ou un auto-injecteur.

4. Dispositif d'injection (1) selon l'une des revendications précédentes, **caractérisé en ce que** la partie distale (13) est liée de façon amovible à la partie proximale (5).

5. Dispositif d'injection (1) selon l'une des revendications précédentes, **caractérisé en ce que** l'écran (15) est retenu de façon mobile contre la partie distale (13).

6. Dispositif d'injection (1) selon l'une des revendications précédentes, **caractérisé en ce qu'**une face avant distale (35) de la partie distale (13) comporte une ouverture de chargement (39) à travers laquelle on peut introduire la pièce de chargement d'injection (3) dans la partie distale (13), la pièce de chargement d'injection (3) pouvant préférentiellement être introduite par rotation dans la partie distale (13), en particulier pouvant être vissée, et/ou la pièce de chargement d'injection (3) pouvant être disposée fixée axialement dans la partie distale (13).

7. Dispositif d'injection (1) selon l'une des revendications précédentes, **caractérisé en ce que** le mécanisme de blocage de l'écran (89) est conçu pour se déverrouiller par introduction de la pièce de chargement d'injection (3) dans la partie distale (13) et pour être verrouillé par une tige de piston (41) affectée à l'entraînement électrique (7).

8. Dispositif d'injection (1) selon l'une des revendications précédentes, **caractérisé en ce que** le disque tournant (91)
a) comporte au moins une surface de glissement (103) sur laquelle peut glisser une structure d'activation (95) de la tige de piston (41), de sorte que le disque tournant (91) soit tourné de la position de déblocage à la position de blocage quand la tige de piston (41) est déplacée axialement dans une direction déterminée.

9. Dispositif d'injection (1) selon l'une des revendications précédentes, **caractérisé en ce que** le dispositif d'injection (1) comporte une butée mobile (77) pour l'écran (15) mobile, l'écran (15) butant préférentiellement sur la butée (77) mobile dans une position d'exposition, dans laquelle on peut réaliser une injection.

10. Pièce de chargement d'injection (3) pour utilisation dans un dispositif d'injection (1) selon l'une des revendications 1 à 9, avec une pièce de chargement d'emballage (17) dans laquelle est contenu un emballage primaire (23), une substance pharmaceutique étant disposée dans l'emballage primaire (23), **caractérisée en ce que**
la pièce de chargement d'injection (3) comporte au moins un cliquet de verrouillage (67) qui est conçu pour coopérer avec l'au moins une surface de verrouillage (99) du disque tournant (91) de la partie distale (13), de façon que le disque tournant (91) soit tourné de la position de blocage en position de déblocage quand la pièce de chargement d'injection (3) est introduite par rotation dans la partie distale (13).

11. Pièce de chargement d'injection (3) selon la revendication 10, **caractérisée en ce que** la pièce de chargement d'emballage (17) comporte un manchon fonctionnel (19) et une pièce de fixation (29), l'emballage primaire (23) étant reçu dans le manchon fonctionnel (19), et l'emballage primaire (23) étant fixé par la pièce de fixation (29) dans le manchon fonctionnel (19).

12. Pièce de chargement d'injection (3) selon l'une des revendications 10 ou 11, **caractérisée en ce que** la pièce de chargement d'injection (3) comporte une pièce de protection d'aiguille (21) liée de façon amovible au manchon fonctionnel (19) et qui cache une aiguille d'injection (25) de l'emballage primaire (23) quand la pièce de protection d'aiguille (21) est liée au manchon fonctionnel (19).

13. Pièce de chargement d'injection (3) selon l'une des revendications 10 à 12, **caractérisée en ce que** la pièce de protection d'aiguille (21) comporte un manchon externe de préhension (31) et un élément de protection élastique verrouillable (33) disposé à l'intérieur du manchon externe de préhension (31), dans lequel l'aiguille d'injection (25) est reçue quand la pièce de protection d'aiguille (21) est liée au manchon fonctionnel (19).

14. Pièce de chargement d'injection (3) selon l'une des revendications 10 à 13, **caractérisée en ce que** la pièce de protection d'aiguille (21) est verrouillée sur le manchon fonctionnel (19), de sorte que la pièce de protection d'aiguille (21) puisse être séparée du manchon fonctionnel (19) quand la pièce de chargement d'injection (3) est disposée dans la partie distale (13).
